(19)

Europäisches
Patentamt
European
Patent Office
Office européen
des brevets

(11) **EP 4 253 609 A1**

(12) **EUROPEAN PATENT APPLICATION**
published in accordance with Art. 153(4) EPC

(43) Date of publication:
**04.10.2023 Bulletin 2023/40**

(21) Application number: **21897098.6**

(22) Date of filing: **25.11.2021**

(51) International Patent Classification (IPC):
*C40B 40/06* [(2006.01)]     *A61K 38/02* [(2006.01)]
*A61K 38/37* [(2006.01)]     *A61K 39/395* [(2006.01)]
*A61K 47/54* [(2017.01)]

(52) Cooperative Patent Classification (CPC):
**A61K 38/02; A61K 38/19; A61K 38/37;
A61K 39/215; A61K 39/385; A61K 39/395;
A61K 47/54; A61K 47/55; A61P 7/00; A61P 31/14;
C07K 19/00; C40B 40/06; G16B 5/00; G16B 15/00;
G16B 15/10;**        (Cont.)

(86) International application number:
**PCT/CN2021/133254**

(87) International publication number:
**WO 2022/111598 (02.06.2022 Gazette 2022/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.11.2020 CN 202011340377**

(71) Applicant: **Assembly Medicine, LLC.
Shanghai, 201203 (CN)**

(72) Inventors:
- **YANG, Fan
Shanghai 201203 (CN)**
- **CHOU, James Jeiwen
Shanghai 201203 (CN)**
- **ZHOU, Liujuan
Shanghai 201203 (CN)**
- **WANG, Yanbing
Shanghai 201203 (CN)**
- **CAO, Chan
Shanghai 201203 (CN)**
- **RUN, Changqing
Shanghai 201203 (CN)**

(74) Representative: **Lavoix
Bayerstraße 83
80335 München (DE)**

Remarks:
The complete document including Reference
Table(s) and the Sequence Listing(s) can be
downloaded from the EPO website

(54) **CONSTRUCTION METHOD FOR AND APPLICATION OF NUCLEIC ACID MULTIMERIZATION-MEDIATED MULTIVALENT PROTEIN DRUG AND VACCINE**

(57) A construction method for and an application of a nucleic acid multimerization-mediated multivalent protein drug and vaccine. Specifically provided is a multimeric complex based on complementary nucleic acid backbones. The complex is a multimer formed by complexing of 3-6 monomers having complementary nucleic acid backbones, wherein each monomer is a polypeptide having a nucleic acid single strand. In the multimer, the nucleic acid single strand of each monomer and the nucleic acid single strands of the other two monomers form double strands by means of base complementation, so as to form complementary nucleic acid backbone structures. Also provided are a pharmaceutical composition containing the multimeric complex, a nucleic acid sequence library used for constructing the multimeric complex, and a method for optimizing complementary nucleic acid backbones. By means of the method, off-the-shelf short-acting protein drugs or antigens can be used to complete multivalent formation of protein drugs or antigens without the need of reconstruction of fusion proteins or chemical modification and cross-linking, thereby improving their half-life and activity, and/or immunogenicity.

EP 4 253 609 A1

**(Cont. next page)**

(52) Cooperative Patent Classification (CPC): (Cont.)
     **G16B 15/30**

**Description**

**Technical field**

The present invention relates to the field of biotechnology drugs, and specifically relates to a construction method for and an application of a nucleic acid multimerization-mediated multivalent protein drug and vaccine.

**Background**

For many biological macromolecules, their aggregation or multivalent state directly affects their activities and half-lives in vivo. For example, the activation of most immune receptors involves the aggregation of receptors on the cell membrane, thereby activating downstream signaling pathways within the cell. Therefore, the ability of natural ligands or antibodies of these receptors to activate receptors can often be significantly improved when they form multivalent or high-valent forms. In addition, some protein drugs with lower molecular weights (MW 40 kDa), such as cytokines, growth hormones, synthetic peptides, etc., have high renal clearance efficiencies and short half-lives in vivo; the molecular weights and half-lives of these protein drugs can also be increased by forming high-valent forms.

Therefore, multivalent formation of proteins is a highly concerned process in the field of biomedicine, and there are many existing methods. However, most chemical crosslinking methods have poor connection specificity and uneven connection of multimers. The most widely used method currently is to express and produce proteins in the form of multivalent fusion proteins by cells, which involves fusing drug functional protein regions with proteins that can form oligomers to form chimeras, such as Fc divalent fusion proteins and GCN4 trivalent fusion proteins, etc. These fusion proteins can form even oligomers, but the cellular expression and activity of fusion proteins are often worse than those of original protein drugs, and the presence of Fc regions brings about the activation of the immune system, induction of cytokine release, and risk of cytotoxicity. Therefore, there is an urgent need to develop a simple, flexible, and efficient method in this field that can form even and highly specific multivalent proteins from validated protein drugs in a non-fusion protein manner.

In addition, multivalent formation of proteins is also of great significance for vaccine development. Firstly, in the design of B cell-based vaccines, activating B cell receptors (BCRs) using viral or bacterial proteins as antigens is a crucial step. Like the immune receptors mentioned above, the effective activation of BCR requires the aggregation of receptors on the cell membrane, so high-valent antigens have an absolute advantage over monomeric antigens in activating B cells. Secondly, high-valent antigens may not necessarily form oligomers from a single antigen; high-valent antigens can contain different proteins in a certain virus or mutations and subtypes of the same protein in different virus strains; in this way, diversified antigen presentation can theoretically induce polyclonal response of B cells in the host immune system, producing a wider range of neutralizing antibodies.

**Summary of the invention**

One purpose of the present invention is to provide an efficient and stable assembly backbone design for n-order nucleic acid oligomers, suitable for the efficient and stable assembly of nucleic acid coupled protein drugs to form multivalent drugs or vaccines.

The second purpose of the present invention is to provide a simple and efficient method for forming multivalent macromolecules from protein drugs for extending the half-life of drugs and increasing drug activity.

The third purpose of the present invention is to provide a simple, flexible, efficient, and modular method for forming multivalent macromolecular complexes of the same or different protein antigens for activating immune cells and improving the immunogenicity of vaccines.

In the first aspect of the present invention, it provides a multimeric complex based on a complementary nucleic acid backbone, wherein the complex is a multimer formed by complexing n monomers having the complementary nucleic acid backbone, wherein each monomer is a polypeptide having a nucleic acid single strand, and n is a positive integer of 3-6; in the multimer, the nucleic acid single strand of each monomer and the nucleic acid single strands of the other two monomers form complementary double strands by means of base complementation, so as to form complementary nucleic acid backbone structures.

In another preferred embodiment, n is 3, 4, 5, or 6.

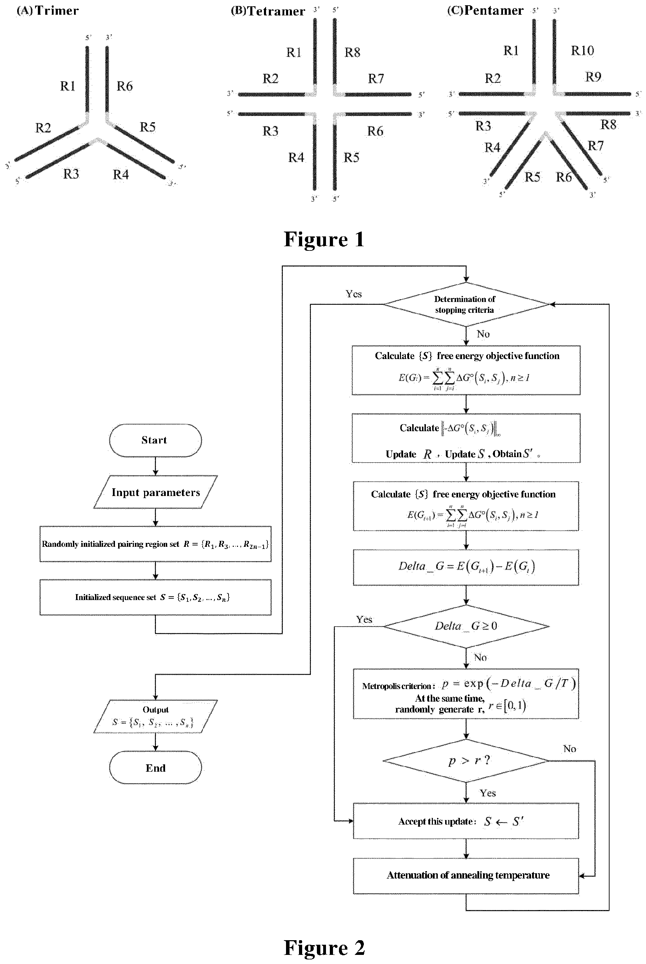

In another preferred embodiment, the complex is a trimer, tetramer, or pentamer, preferably with a structure as shown in Figure 1.

In another preferred embodiment, the monomer has a structure of formula I:

$$Z1\text{-}W \qquad (I)$$

wherein,

Z1 is a polypeptide moiety;
W is a nucleic acid single strand sequence; and
"-" is a linker or bond.

In another preferred embodiment, "-" is a covalent bond.
In another preferred embodiment, the nucleic acid sequence is selected from the group consisting of: left-handed nucleic acid, peptide nucleic acid, locked nucleic acid, thio-modified nucleic acid, 2'-fluoro modified nucleic acid, 5-hydroxymethylcytosine nucleic acid, phosphorodiamidate morpholino nucleic acid, and combinations thereof;
In another preferred embodiment, in the multimer, the Z1 of each monomer is the same or different.
In another preferred embodiment, in the multimer, the W of each monomer is different.
In another preferred embodiment, the monomer has a structure of formula II:

$$D\text{-}[L\text{-}W]m \qquad (II)$$

wherein,

D is a protein drug element moiety;
W is a nucleic acid sequence;
L is not present or a linker;
"-" is a covalent bond; and
m is 1, 2, or 3.

In another preferred embodiment, m is 1.
In another preferred embodiment, the monomer has a structure of formula III:

$$A\text{-}[L\text{-}W]m \qquad (III)$$

wherein,

A is a peptide antigen element moiety;
W is a nucleic acid sequence;
L is not present or a linker;
"-" is a covalent bond; and
m is 1, 2, or 3.

In another preferred embodiment, m is 1.
In another preferred embodiment, the nucleic acid sequence W has the structure shown in formula 1:

$$X1\text{-}R1\text{-}X2\text{-}R2\text{-}X3 \qquad (1)$$

wherein,

R1 is a complementary base pairing region 1;
R2 is a complementary base pairing region 2;
Each of X1, X2, and X3 is independently not present or redundant nucleic acids; and
"-" is a bond.

In another preferred embodiment, each of R1 and R2 is independently 10-20 bases, preferably 14-16 bases in length.
In another preferred embodiment, X1 is 0-5 bases in length.
In another preferred embodiment, X3 is 0-5 bases in length.
In another preferred embodiment, X2 is 0-3 bases in length.
In another preferred embodiment, the sequence of X2 is selected from the group consisting of: A, AA, AGA and AAA.
In another preferred embodiment, the R1 of each monomer forms a complementary base pairing structure with the R2 of the left neighbor (or left side) monomer; while the R2 forms a complementary base pairing structure with the R1 of the right neighbor (or right side) monomer.

In another preferred embodiment, the monomer sequence is any sequence or a sequence set thereof selected from the nucleic acid single strand sequences as shown in SEQ ID Nos: 1-60 (see Table 9-1) that form a trimer complex based on the complementary nucleic acid backbone.

In another preferred embodiment, the monomer sequence is any sequence or a sequence set thereof selected from the nucleic acid single strand sequences as shown in SEQ ID Nos: 61-140 (see Table 9-2) that form a tetramer complex based on the complementary nucleic acid backbone.

In another preferred embodiment, the monomer sequence is any sequence or a sequence set thereof selected from the nucleic acid single strand sequences as shown in SEQ ID Nos: 141-240 (see Table 9-3) that forms a pentamer complex based on the complementary nucleic acid backbone.

In another preferred embodiment, the monomer sequence is a phosphorodiamidate morpholino nucleic acid.

In another preferred embodiment, the monomer sequence is any sequence or a sequence set thereof selected from the nucleic acid single strand sequences as shown in SEQ ID Nos: 275-278 that forms a tetramer complex based on the complementary nucleic acid backbone.

In the second aspect of the present invention, it provides a pharmaceutical composition comprising:

(a) the multimeric complex based on the complementary nucleic acid backbone according to the first aspect; and
(b) a pharmaceutically acceptable carrier.

In another preferred embodiment, the pharmaceutical composition comprises a vaccine composition.

In another preferred embodiment, the pharmaceutical composition comprises a therapeutic and/or prophylatic pharmaceutical composition.

In another preferred embodiment, the multimeric complex comprises a trimer complex, a tetramer complex, and a pentamer complex.

In the third aspect of the present invention, it provides a nucleic acid sequence library, which comprises a nucleic acid sequence for forming the multimeric complex based on the complementary nucleic acid backbone according to the first aspect.

In another preferred embodiment, the nucleic acid sequence comprises:

(a) a nucleic acid sequence for forming a trimer complex based on the complementary nucleic acid backbone;
(b) a nucleic acid sequence for forming a tetramer complex based on the complementary nucleic acid backbone; and/or
(c) a nucleic acid sequence for forming a pentamer complex based on the complementary nucleic acid backbone.

In another preferred embodiment, the nucleic acid sequence W has the structure shown in formula 1:

$$X1\text{-}R1\text{-}X2\text{-}R2\text{-}X3 \qquad (1)$$

wherein,

R1 is the complementary base pairing region 1;
R2 is the complementary base pairing region 2;
Each of X1, X2, and X3 is independently not present or redundant nucleic acids; and
"-" is a bond.

In the fourth aspect of the present invention, it provides use of the nucleic acid sequence library according to the third aspect in the manufacture of the multimeric complex according to the first aspect or a pharmaceutical composition comprising the multimeric complex.

In the fifth aspect of the present invention, it provides a method of determining a nucleic acid single strand sequence for forming a multimeric complex based on a complementary nucleic acid backbone, comprising steps of:

(a) setting annealing algorithm parameters:

setting the initial annealing temperature, annealing termination temperature, and annealing temperature attenuation coefficient $\Delta T$;
setting optimized constraint parameters:

①the number n of the nucleic acid single strand, preferably a positive integer of 3-6;

②the length $L$ of the pairing sequence, preferably the $L$ is of 12-16 bases;

③the dissociation temperature threshold $T_m$ of the pairing region;

④the free energy threshold $\Delta G_S^\circ$ of the specific pairing region sequence;

⑤the free energy threshold $\Delta G_{NS}^\circ$ of the non-specific pairing;

⑥the connecting element X2, preferably A, AA, and AAA;

⑦the dissociation temperature threshold $T_{m\text{-}H}$ of the secondary structure (hairpin);

⑧the CG proportion $P_{CG}$ in the pairing sequence, preferably the range of $P_{CG}$ is [0.4,0.6);

⑨optionally, for n=4, using a symmetric sequence to initialize a sequence set $S = \{S_1, S_2, \ldots , S_n\}$ according to the above parameters;

(b) calculating the objective function value $E_0$ of the set $S$ of the previous step, that is, calculating the sum of the non-specific pairing free energies ( $\Delta G_{NS}^\circ$ ) between sequences and of the sequence itself, while obtaining the non-specific pairing free energy matrix $C_{n \times n}$, searching the $S_i$ and $S_j (1 \leq i \leq n, 1 \leq j \leq n)$ corresponding to the minimum value in the upper triangular matrix thereof, randomly selecting $S_i$ or $S_j$ for an updated operation according to the non-specific pairing free energy of the $S_i$ and $S_j$ $\Delta G_{NS}^\circ(S_i, S_j)$ , and then obtaining a new nucleic acid sequence, thereby obtaining a updated sequence set $S'$;

(c) determining whether the sequences in the set $S'$ of the previous step meet the optimized constraint parameter conditions set in step (a), verifying the following parameters, including the dissociation temperature $T_m$ of the specific pairing region, the free energy $\Delta G_S^\circ$ of the specific pairing region sequence, the dissociation temperature $T_{m\text{-}H}$ of the secondary structure and the CG proportion $P_{CG}$. If the above parameters meet the constraint conditions, the step (d) is proceeded; otherwise, the step (c) is repeated. If the step (b) is performed 15 times continuously at a certain annealing temperature without obtaining the $S'$ that meets the conditions, then the set S becomes the set $S'$ and the next step is proceeded to prevent a dead cycle;

(d) calculating the objective function value $E_1$ of the set $S'$ of the previous step, and comparing $E_0$ with $E_1$. If $E_1 \geq E_0$, it indicates that the non-specific pairing free energy has been optimized, and the sequence set $S'$ becomes the sequence set $S$. If $E_1 < E_0$, it indicates that the non-specific pairing free energy has not been optimized, and in this case, it is necessary to determine whether to accept the set $S'$ as $S$ according to the Metropolis criterion; and

(e) the annealing temperature is attenuated according to the attenuation coefficient $\Delta T$ set in the step (a), and the steps (b), (c), and (d) are repeated for the S of the previous step, which is the Monte Carlo-based annealing algorithm, until the annealing temperature reaches the annealing termination temperature. The $S = \{S_1, S_2, \ldots, S_n\}$ of the previous step becomes the nucleic acid single strand sequence for forming the multimeric complex based on the complementary nucleic acid backbone.

In another preferred embodiment, in step (a) setting annealing algorithm parameters, it comprises:

for example, setting the initial annealing temperature $T_o = 50°C \pm 2°C$, the annealing termination temperature $T_f = 0.12°C \pm 0.02°C$, and the annealing temperature attenuation coefficient $\Delta T$ depends on the situation, usually $0.98 \pm 0.01$;

setting optimized constraint parameters:

①the number n of the nucleic acid single strand is a positive integer of 3-6,

②the length $L$ of the pairing sequence depends on the situation (preferably the L is of 12-16 bases),

③ the dissociation temperature threshold $T_m$ of the pairing region is determined by the length of the pairing sequence (e.g., when $L = 14$ bases, $T_m > 50°C$; when $L = 16$ bases, $T_m > 52°C$),

④the free energy threshold $\Delta G_S^\circ$ of the specific pairing region sequence is determined by the length of the pairing sequence (preferably, when $L = 14$ bases, $\Delta G_S^\circ < -27$ kcal/mol; when L = 16 bases, $\Delta G_S^\circ < -29$ kcal/mol),

⑤the free energy threshold $\Delta G_{NS}^\circ$ of the non-specific pairing is determined by the sequence length (preferably,

$$\Delta G_{NS}^{\circ} > -7 \text{ kcal/mol),}$$

⑥the connecting element X2 depends on the situation (can be A, AA, and AAA, etc.),

⑦the dissociation temperature threshold $T_{m-H}$ of the secondary structure (hairpin) depends on the situation (preferably, $T_{m-H} < 40°C \pm 2°C$),

⑧the range of the CG proportion $P_{CG}$ in the pairing sequence is [0.4,0.6],

⑨specifically, for n=4, a symmetric sequence can be used to initialize the sequence $S = \{S_1, S_2, \ldots, S_n\}$ according to the above parameters.

In another preferred embodiment, each nucleic acid single strand sequence W has the structure shown in formula 1:

X1-R1-X2-R2-X3            (1)

wherein,

R1 is the complementary base pairing region 1;
R2 is the complementary base pairing region 2;
Each of X1, X2, and X3 independently is not present or redundant nucleic acids; and
"-" is a bond.

In another preferred embodiment, in the step (d), the optimized set is a set that satisfies following conditions:

(C1) the free energy ( $\Delta G_S^{\circ}$ ) of the DNA double strand structure formed by the target pairing is smaller or smallest in the complementary nucleic acid backbone structure; and

(C2) the $\Delta G_S^{\circ}$ of the non-target pairing is larger or the largest in the complementary nucleic acid backbone structure.

In another preferred embodiment, in the step (d), the optimized set also satisfies following conditions:
(C3) the pairing dissociation temperature of the R1 and R2 regions $T_m > 50°C$ (when $L$ = 14 bases).

In another preferred embodiment, in the step (c), the free energy ( $\Delta G_S^{\circ}$ ) of the DNA oligomer (i.e., the complementary nucleic acid backbone structure) is calculated using the nearest neighbor method.

In another preferred embodiment, in the step (c), the DNA oligomer (i.e., the complementary nucleic acid backbone structure) is decomposed into 10 different nearest neighbor pairing interactions, which are: AA/TT; AT/TA; TA/AT; CA/GT; GT/CA; CT/GA; GA/CT; CG/GC; GC/CG; and GG/CC; and the corresponding $\Delta G^{\circ}$ value is calculated and obtained respectively based on the enthalpy ( $\Delta H^{\circ}$ ) and entropy ( $\Delta S^{\circ}$ ) of these pairing interactions; then, the free energies of the pairing interactions included in the complementary nucleic acid backbone structure are merged (or summed) to obtain the free energy of the complementary nucleic acid backbone structure.

In another preferred embodiment, the method comprises repeating the steps (b), (c), and (d) for multiple times (i.e., performing n1 iterations) to obtain the global optimal solution during the iteration process.

In another preferred embodiment, during the iteration process, a poor solution is limitedly accepted according to the Metropolis criterion, and the probability of accepting the poor solution is gradually approaching zero, so as to find the global optimal solution at all possible when the algorithm terminates.

In another preferred embodiment, the following optimized objective function is used for the iteration of the simulated annealing algorithm to optimize the free energy of the non-target pairing region:

$$E = \sum_{i=1}^{n} \sum_{j=i}^{n} -\Delta G^{\circ}\left(S_i,\ S_j\right),\ n \geq 1$$

$\Delta G^{\circ}(S_i, S_j)$ is the free energy of the non-target pairing between $S_i$ sequence and $S_j$ sequence, and $\sum_{i=1}^{n} \sum_{j=i}^{n} \Delta G^{\circ}\left(S_i,\ S_j\right)$ is the sum of the free energies of non-target pairing between all sequences, with a negative value; wherein the larger the negative value, the more beneficial it is to reduce the non-target pairing.

In the sixth aspect of the present invention, it provides a nucleic acid single strand sequence set for forming a multimeric complex based on a complementary nucleic acid backbone, which is determined using the method of the fifth aspect.

In another preferred embodiment, the set is selected from the group consisting of:
(S 1) a nucleic acid single strand sequence for forming a trimer complex based on the complementary nucleic acid backbone:

Table 9-1

| Sequence set 3-1 numbering | Optimized sequence | SEQ ID NO: |
|---|---|---|
| $S_1$ | ACACCTGGTTGTTGGATAAATCGTTGAAG GCTAGGA | 1 |
| $S_2$ | ATCCTAGCCTTCAACGAAAAAACTAGAGT CCGCCGA | 2 |
| $S_3$ | ATCGGCGGACTCTAGTTAAAATCCAACAA CCAGGTG | 3 |
| Sequence set 3-2 numbering | Optimized sequence | |
| $S_1$ | ATGCGTTGAGTTCCAGTAAAGGCAACATC ACCACAT | 4 |
| $S_2$ | AATGTGGTGATGTTGCCAAATCTGAATCC TCGTGCT | 5 |
| $S_3$ | AAGCACGAGGATTCAGAAAAACTGGAAC TCAACGCA | 6 |
| Sequence set 3-3 numbering | Optimized sequence | |
| $S_1$ | ATTCCAATCGTCCTGTGAAAAGTTCCGCT CTGAGTT | 7 |
| $S_2$ | AAACTCAGAGCGGAACTAAACTGGCAGA TGGATGAA | 8 |
| $S_3$ | ATTCATCCATCTGCCAGAAACACAGGACG ATTGGAA | 9 |
| Sequence set 3-4 numbering | Optimized sequence | |
| $S_1$ | ACGAGGCAAGTTCTGTGAAAATGACTACC AGGTCCG | 10 |
| $S_2$ | ACGGACCTGGTAGTCATAAAATCCACTGA CGCTGAA | 11 |
| $S_3$ | ATTCAGCGTCAGTGGATAAACACAGAACT TGCCTCG | 12 |
| Sequence set 3-5 numbering | Optimized sequence | |
| $S_1$ | ATAGTTCGTTGCTCGGAAAAGGCATTGAG AGGACCT | 13 |
| $S_2$ | AAGGTCCTCTCAATGCCAAAATGGTGATG TCGCTTG | 14 |
| $S_3$ | ACAAGCGACATCACCATAAATCCGAGCAA CGAACTA | 15 |

(continued)

| Sequence set 3-6 numbering | Optimized sequence | |
|---|---|---|
| $S_1$ | <u>A</u>GTCGTGTGCTTCCAAG<u>AAA</u>TAGCCAGGTGAGGACT | 16 |
| $S_2$ | <u>A</u>AGTCCTCACCTGGCTA<u>AAA</u>AACAGCGGAGTGTCAT | 17 |
| $S_3$ | <u>A</u>ATGACACTCCGCTGTT<u>AAA</u>CTTGGAAGCACACGAC | 18 |
| Sequence set 3-7 numbering | Optimized sequence | |
| $S_1$ | <u>A</u>ACGCATCGCTTGATAG<u>AAA</u>AGAGGAGCACGGTTAT | 19 |
| $S_2$ | <u>A</u>ATAACCGTGCTCCTCT<u>AAA</u>GTAGGCAATCCACCAT | 20 |
| $S_3$ | <u>A</u>ATGGTGGATTGCCTAC<u>AAA</u>CTATCAAGCGATGCGT | 21 |
| Sequence set 3-8 numbering | Optimized sequence | |
| $S_1$ | <u>A</u>GTCGTTCCACCGAACA<u>AAA</u>TGGCTCTGGTCATTGA | 22 |
| $S_2$ | <u>A</u>TCAATGACCAGAGCCA<u>AAAA</u>ATCGCACATCTCAGG | 23 |
| $S_3$ | <u>A</u>CCTGAGATGTGCGATT<u>AAA</u>TGTTCGGTGGAACGAC | 24 |
| Sequence set 3-9 numbering | Optimized sequence | |
| $S_1$ | <u>A</u>GCGGAGTGACCATAGT<u>AAA</u>AGGCAGGACATTGTTC | 25 |
| $S_2$ | <u>A</u>GAACAATGTCCTGCCT<u>AAA</u>GTGCTCGTCGTGAAGA | 26 |
| $S_3$ | <u>A</u>TCTTCACGACGAGCAC<u>AAA</u>CTATGGTCACTCCGC | 27 |
| Sequence set 3-10 numbering | Optimized sequence | |
| $S_1$ | <u>A</u>ATTGGACCGCTCTACT<u>AAA</u>ATGGCACCACAGTCAA | 28 |
| $S_2$ | <u>A</u>TTGACTGTGGTGCCAT<u>AAA</u>CAGGCTATCAGCATCC | 29 |
| $S_3$ | <u>A</u>GGATGCTGATAGCCTG<u>AAA</u>AGTAGAGCGGTCCAAT | 30 |

(continued)

| Sequence set 3-11 numbering | Optimized sequence | |
|---|---|---|
| $S_1$ | <u>A</u>CCATTGAGCCAGTGAT<u>AAA</u>AACCGTTGT GAGTTGC | 31 |
| $S_2$ | <u>A</u>GCAACTCACAACGGT<u>AAA</u>TCGCACACC TGTCGTA | 32 |
| $S_3$ | <u>A</u>TACGACAGGTGTGCGA<u>AAA</u>ATCACTGGC TCAATGG | 33 |
| Sequence set 3-12 numbering | Optimized sequence | |
| $S_1$ | <u>A</u>AGTGAAGAAGCAGCCT<u>AAA</u>GTTGTCATC GCACACC | 34 |
| $S_1 S_2$ | <u>A</u>GGTGTGCGATGACAAC<u>AAA</u>ATGTCGTAA CCGTGGA | 35 |
| $S_3$ | <u>A</u>TCCACGGTTACGACAT<u>AAA</u>AGGCTGCTT CTTCACT | 36 |
| Sequence set 3-13 numbering | Optimized sequence | |
| $S_1$ | <u>A</u>ATAGCGTCTTGAGCCT<u>AAA</u>TGGAGGACA TACCGAC | 37 |
| $S_2$ | <u>A</u>GTCGGTATGTCCTCCA<u>AAA</u>GGTCACAGT TGCTGCT | 38 |
| $S_3$ | <u>A</u>AGCAGCAACTGTGACC<u>AAA</u>AGGCTCAA GACGCTAT | 39 |
| Sequence set 3-14 numbering | Optimized sequence | |
| $S_1$ | <u>A</u>TGCCGTGTTCAGATTC<u>AAA</u>TGTGCGTCT GGATTGA | 40 |
| $S_2$ | <u>A</u>TCAATCCAGACGCACA<u>AAA</u>AGACAGGT GGTCCGAT | 41 |
| $S_3$ | <u>A</u>ATCGGACCACCTGTCT<u>AAA</u>GAATCTGAA CACGGCA | 42 |
| Sequence set 3-15 numbering | Optimized sequence | |
| $S_1$ | <u>A</u>TTCAGGACAGCGTCAT<u>AAA</u>CCGACTGG AGCAACT | 43 |
| $S_2$ | <u>A</u>AGTTGCTCCAGTCGGT<u>AAA</u>GATGCCTTC GTGTGAG | 44 |
| $S_3$ | <u>A</u>CTCACACGAAGGCATC<u>AAA</u>ATGACGCTG TCCTGAA | 45 |

(continued)

| Sequence set 3-16 numbering | Optimized sequence | |
|---|---|---|
| $S_1$ | AGCAGCCAAGGTTATCTAAACAATGACAC GGAGGAT | 46 |
| $S_2$ | AATCCTCCGTGTCATTGAAAGTGATTCGC ACCAGAC | 47 |
| $S_3$ | AGTCTGGTGCGAATCACAAAAGATAACCT TGGCTGC | 48 |
| Sequence set 3-17 numbering | Optimized sequence | |
| $S_1$ | ACCACCGTGTATGACCTAAAAGTGACAGC ACATCGC | 49 |
| $S_2$ | AGCGATGTGCTGTCACTAAAACAGGCTCT ACGAGGA | 50 |
| $S_3$ | ATCCTCGTAGAGCCTGTAAAAGGTCATAC ACGGTGG | 51 |
| Sequence set 3-18 numbering | Optimized sequence | |
| $S_1$ | AACTACGGAGCGAAGATAAATCCTGACCA ACTTGCT | 52 |
| $S_2$ | AAGCAAGTTGGTCAGGAAAAGACTGGCT GAACACGA | 53 |
| $S_3$ | ATCGTGTTCAGCCAGTCAAAATCTTCGCT CCGTAGT | 54 |
| Sequence set 3-19 numbering | Optimized sequence | |
| $S_1$ | AGTTCCTGATCCAGCCTAAACATCCTTGTC TTGCCA | 55 |
| $S_2$ | ATGGCAAGACAAGGATGAAACACGACCG CTTAGAAG | 56 |
| $S_3$ | ACTTCTAAGCGGTCGTGAAAAGGCTGGAT CAGGAAC | 57 |
| Sequence set 3-20 numbering | Optimized sequence | |
| $S_1$ | ATATCGCACTCCAGCATAAACCGTGTGAA CATCAGG | 58 |
| $S_2$ | ACCTGATGTTCACACGGAAAAGCCTACGA GACTTGG | 59 |
| $S_3$ | ACCAAGTCTCGTAGGCTAAAATGCTGGAG TGCGATA | 60 |

(S2) a nucleic acid single strand sequence for forming a tetramer complex based on the complementary nucleic acid backbone:

Table 9-2

| Sequence set 4-1 numbering | Optimized sequence | SEQ ID NO: |
|---|---|---|
| $S_1$ | <u>A</u>AGCGTCGTGAATCC<u>AAA</u>TGAGCCTGCCAATG | 61 |
| $S_2$ | <u>A</u>CATTGGCAGGCTCA<u>AAA</u>CCGAAGTCAACGCT | 62 |
| $S_3$ | <u>A</u>AGCGTTGACTTCGG<u>AAA</u>CTATGGACGGCGA | 63 |
| $S_4$ | <u>A</u>TCGCCGTCCATAGT<u>AAA</u>GGATTCACGACGCT | 64 |
| Sequence set 4-2 numbering | Optimized sequence | |
| $S_1$ | <u>A</u>ATGGCGAGCAATCC<u>AAA</u>TGAGCCTGGACCAA | 65 |
| $S_2$ | <u>A</u>TTGGTCCAGGCTCA<u>AAA</u>CCGAACGCTGTGAT | 66 |
| $S_3$ | <u>A</u>ATCACAGCGTTCGG<u>AAA</u>CTATCGTGCGGCA | 67 |
| $S_4$ | <u>A</u>TGCCGCACGATAGT<u>AAA</u>GGATTGCTCGCCAT | 68 |
| Sequence set 4-3 numbering | Optimized sequence | |
| $S_1$ | <u>A</u>TGACCACGCAATCC<u>AAA</u>TGAGCCAACCTCCA | 69 |
| $S_2$ | <u>A</u>TGGAGGTTGGCTCA<u>AAA</u>CCGAACAGCAGCTT | 70 |
| $S_3$ | <u>A</u>AAGCTGCTGTTCGG<u>AAA</u>CTATCTGCCGCCT | 71 |
| $S_4$ | <u>A</u>AGGCGGCAGATAGT<u>AAA</u>GGATTGCGTGGTCA | 72 |
| Sequence set 4-4 numbering | Optimized sequence | |
| $S_1$ | <u>A</u>TGTCGCACCAATCC<u>AAA</u>TGAGCAAGCCTCGT | 73 |
| $S_2$ | <u>A</u>ACGAGGCTTGCTCA<u>AAA</u>CCGAACGCTGTCAT | 74 |
| $S_3$ | <u>A</u>ATGACAGCGTTCGG<u>AAA</u>CTATGTGGCGGCA | 75 |
| $S_4$ | <u>A</u>TGCCGCCACATAGT<u>AAA</u>GGATTGGTGCGACA | 76 |

(continued)

| Sequence set 4-5 numbering | Optimized sequence | |
|---|---|---|
| $S_1$ | ATGCTGGCACAATCCAAATGAGCGACG AGGTT | 77 |
| $S_2$ | AAACCTCGTCGCTCAAAACCGAAGTGC CAGTT | 78 |
| $S_3$ | AAACTGGCACTTCGGAAAACTATGAGG CGGCT | 79 |
| $S_4$ | AAGCCGCCTCATAGTAAAGGATTGTGC CAGCA | 80 |
| Sequence set 4-6 numbering | Optimized sequence | |
| $S_1$ | ATGTCGCACCAATCCAAATGAGCAGGT TGGCA | 81 |
| $S_2$ | ATGCCAACCTGCTCAAAACCGAACGCT GTCAA | 82 |
| $S_3$ | ATTGACAGCGTTCGGAAAACTATCAGC CGCCT | 83 |
| $S_4$ | AAGGCGGCTGATAGTAAAGGATTGGTG CGACA | 84 |
| Sequence set 4-7 numbering | Optimized sequence | |
| $S_1$ | ATGTGGTCGCAATCCAAATGAGCACCT GCCAA | 85 |
| $S_2$ | ATTGGCAGGTGCTCAAAACCGAACGTG ACGAT | 86 |
| $S_3$ | AATCGTCACGTTCGGAAAACTATCAAC GCCGC | 87 |
| $S_4$ | AGCGGCGTTGATAGTAAAGGATTGCGA CCACA | 88 |
| Sequence set 4-8 numbering | Optimized sequence | |
| $S_1$ | AAGCGTCGTCAATCCAAATGAGCACGG CAATG | 89 |
| $S_2$ | ACATTGCCGTGCTCAAAACCGAAGTGA ACGCT | 90 |
| $S_3$ | AAGCGTTCACTTCGGAAAACTATGGCT CGCCT | 91 |
| $S_4$ | AAGGCGAGCCATAGTAAAGGATTGACG ACGCT | 92 |

(continued)

| Sequence set 4-9 numbering | Optimized sequence | |
|---|---|---|
| $S_1$ | ATGTGGCGACAATCCAAATGAGCAAGCCTCCA | 93 |
| $S_2$ | ATGGAGGCTTGCTCAAAACCGAAGACGCTGTT | 94 |
| $S_3$ | AAACAGCGTCTTCGGAAAACTATCGTGCGGCA | 95 |
| $S_4$ | ATGCCGCACGATAGTAAAGGATTGTCGCCACA | 96 |
| Sequence set 4-10 numbering | Optimized sequence | |
| $S_1$ | ATGCTGCCACAATCCAAATGAGCCTGGAACCA | 97 |
| $S_2$ | ATGGTTCCAGGCTCAAAACCGAACGCAGTCAT | 98 |
| $S_3$ | AATGACTGCGTTCGGAAAACTATCGCCGCTCT | 99 |
| $S_4$ | AAGAGCGGCGATAGTAAAGGATTGTGGCAGCA | 100 |
| Sequence set 4-11 numbering | Optimized sequence | |
| $S_1$ | ATGCGTCGTCAATCCAAATGAGCTTGGCAAGG | 101 |
| $S_2$ | ACCTTGCCAAGCTCAAAACCGAACGTGCTGTT | 102 |
| $S_3$ | AAACAGCACGTTCGGAAAACTATGGAGCGGCT | 103 |
| $S_4$ | AAGCCGCTCCATAGTAAAGGATTGACGACGCA | 104 |
| Sequence set 4-12 numbering | Optimized sequence | |
| $S_1$ | AACTGCCAGCAATCCAAATGAGCCTCGTTCCA | 105 |
| $S_2$ | ATGGAACGAGGCTCAAAACCGAAGTTGGCAGT | 106 |
| $S_3$ | AACTGCCAACTTCGGAAAACTATCGCCGCTTG | 107 |
| $S_4$ | ACAAGCGGCGATAGTAAAGGATTGCTGGCAGT | 108 |

(continued)

| Sequence set 4-13 numbering | Optimized sequence | |
|---|---|---|
| $S_1$ | ATGCGTCGTCAATCCAAATGAGCCTCC AGGTT | 109 |
| $S_2$ | AAACCTGGAGGCTCAAAACCGAATGAC ACGCT | 110 |
| $S_3$ | AAGCGTGTCATTCGGAAAACTATGGCG GCAGT | 111 |
| $S_4$ | AACTGCCGCCATAGTAAAGGATTGACG ACGCA | 112 |
| Sequence set 4-14 numbering | Optimized sequence | |
| $S_1$ | AAGCGTCGTGAATCCAAATGAGCCATC GTCCA | 113 |
| $S_2$ | ATGGACGATGGCTCAAAACCGAATGTG CTGGT | 114 |
| $S_3$ | AACCAGCACATTCGGAAAACTATGCGG CAACC | 115 |
| $S_4$ | AGGTTGCCGCATAGTAAAGGATTCACG ACGCT | 116 |
| Sequence set 4-15 numbering | Optimized sequence | |
| $S_1$ | ATTGCCAGGATGCTGAATCACGGTCGG ACA | 117 |
| $S_2$ | ATGTCCGACCGTGATAGTCGCAGAAGG CAT | 118 |
| $S_3$ | AATGCCTTCTGCGACATAGTACAACGC CGC | 119 |
| $S_4$ | AGCGGCGTTGTACTAACAGCATCCTGG CAA | 120 |
| Sequence set 4-16 numbering | Optimized sequence | |
| $S_1$ | AGGCGATCACAATCCAAATGAGCGTGT TACGG | 121 |
| $S_2$ | ACCGTAACACGCTCAAAACCGAAGTGC CAATT | 122 |
| $S_3$ | AAATTGGCACTTCGGAAAACTATGCGG CTGCT | 123 |
| $S_4$ | AAGCAGCCGCATAGTAAAGGATTGTGA TCGCC | 124 |

(continued)

| Sequence set 4-17 numbering | Optimized sequence | |
|---|---|---|
| $S_1$ | ATGGTCCAACACGCTAAGCCTCACCGTCTT | 125 |
| $S_2$ | AAAGACGGTGAGGCTATCGCACAACCTGGT | 126 |
| $S_3$ | AACCAGGTTGTGCGAATCGGAGTGGCAGAA | 127 |
| $S_4$ | ATTCTGCCACTCCGAAAGCGTGTTGGACCA | 128 |
| Sequence set 4-18 numbering | Optimized sequence | |
| $S_1$ | AACCTTGGTGTGCGAAACTCCTGGCAGCAA | 129 |
| $S_2$ | ATTGCTGCCAGGAGTAAGCGTGTGGTTCCA | 130 |
| $S_3$ | ATGGAACCACACGCTATGAGGACCGTCGTT | 131 |
| $S_4$ | AAACGACGGTCCTCAATCGCACACCAAGGT | 132 |
| Sequence set 4-19 numbering | Optimized sequence | |
| $S_1$ | ATGCCAAGTCCGAGAATGCTGCGAACTGGT | 133 |
| $S_2$ | AACCAGTTCGCAGCAAAGAGCCTGAACCGT | 134 |
| $S_3$ | AACGGTTCAGGCTCTAACGACGCTTGACCA | 135 |
| $S_4$ | ATGGTCAAGCGTCGTATCTCGGACTTGGCA | 136 |
| Sequence set 4-20 numbering | Optimized sequence | |
| $S_1$ | AAGCAGCCTCGTTGAATCGCCAAGACACCT | 137 |
| $S_2$ | AAGGTGTCTTGGCGAAAGTTGCTCCGACGA | 138 |
| $S_3$ | ATCGTCGGAGCAACTAAGCGGTTCTGTGGA | 139 |
| $S_4$ | ATCCACAGAACCGCTATCAACGAGGCTGCT | 140 |

(S3) a nucleic acid single strand sequence for forming a pentamer complex based on the complementary nucleic acid backbone:

Table 9-3

| Sequence set 5-1 numbering | Optimized sequence | SEQ ID NO: |
|---|---|---|
| $S_1$ | ATCAGGCGACCTCTTAAAACCACCATCGT TGC | 141 |
| $S_2$ | AGCAACGATGGTGGTAAAAATCCAAATGA GCGTGTTACGG | 142 |
| $S_3$ | ACCGTAACACGCTCAAAACCGAAGTGCCA ATT | 143 |
| $S_4$ | AAATTGGCACTTCGGAAAACTATGCGGCT GCT | 144 |
| $S_5$ | AAGCAGCCGCATAGTAAAGGATTAAAAA GAGGTCGCCTGA | 145 |
| Sequence set 5-2 numbering | Optimized sequence | |
| $S_1$ | AGGCGACGATGTCTTAAAACCTGGTTGCT GGA | 146 |
| $S_2$ | ATCCAGCAACCAGGTAAAAATCCAAATGA GCGTGTTACGG | 147 |
| $S_3$ | ACCGTAACACGCTCAAAACCGAAGTGCCA ATT | 148 |
| $S_4$ | AAATTGGCACTTCGGAAAACTATGCGGCT GCT | 149 |
| $S_5$ | AAGCAGCCGCATAGTAAAGGATTAAAAA GACATCGTCGCC | 150 |
| Sequence set 5-3 numbering | Optimized sequence | |
| $S_1$ | ATGGAACCTGGTGCTAAATGCTCGCCTGT CAA | 151 |
| $S_2$ | ATTGACAGGCGAGCAAAAAATCCAAATG AGCGTGTTACGG | 152 |
| $S_3$ | ACCGTAACACGCTCAAAACCGAAGTGCCA ATT | 153 |
| $S_4$ | AAATTGGCACTTCGGAAAACTATGCGGCT GCT | 154 |
| $S_5$ | AAGCAGCCGCATAGTAAAGGATTAAAAG CACCAGGTTCCA | 155 |
| Sequence set 5-4 numbering | Optimized sequence | |
| $S_1$ | ATGGTCAGGCGACTTAAAAGGACGAGGTT GCT | 156 |

17

(continued)

| Sequence set 5-4 numbering | Optimized sequence | |
|---|---|---|
| $S_2$ | AAGCAACCTCGTCCTAAAAATCCAAATGAGCGTGTTACGG | 157 |
| $S_3$ | ACCGTAACACGCTCAAAACCGAAGTGCCAATT | 158 |
| $S_4$ | AAATTGGCACTTCGGAAACTATGCGGCTGCT | 159 |
| $S_5$ | AAGCAGCCGCATAGTAAAGGATTAAAAAGTCGCCTGACCA | 160 |
| Sequence set 5-5 numbering | Optimized sequence | |
| $S_1$ | ATGCTGGACCACCTTAAATCAGATGGAGGCGA | 161 |
| $S_2$ | ATCGCCTCCATCTGAAAAAATCCAAATGAGCGTGTTACGG | 162 |
| $S_3$ | ACCGTAACACGCTCAAAACCGAAGTGCCAATT | 163 |
| $S_4$ | AAATTGGCACTTCGGAAACTATGCGGCTGCT | 164 |
| $S_5$ | AAGCAGCCGCATAGTAAAGGATTAAAAAGGTGGTCCAGCA | 165 |
| Sequence set 5-6 numbering | Optimized sequence | |
| $S_1$ | AAACGTCCAGGAGCTAAATCTCGTCGCCTGAA | 166 |
| $S_2$ | ATTCAGGCGACGAGAAAAAATCCAAATGAGCGTGTTACGG | 167 |
| $S_3$ | ACCGTAACACGCTCAAAACCGAAGTGCCAATT | 168 |
| $S_4$ | AAATTGGCACTTCGGAAACTATGCGGCTGCT | 169 |
| $S_5$ | AAGCAGCCGCATAGTAAAGGATTAAAAGCTCCTGGACGTT | 170 |
| Sequence set 5-7 numbering | Optimized sequence | |
| $S_1$ | ACCACGACCATTGCTAAAACTTCAGGCGACG | 171 |
| $S_2$ | ACGTCGCCTGAAGTTAAAAATCCAAATGAGCGTGTTACGG | 172 |

(continued)

| Sequence set 5-7 numbering | Optimized sequence | |
|---|---|---|
| $S_3$ | ACCGTAACACGCTCAAAACCGAAGTGCCAATT | 173 |
| $S_4$ | AAATTGGCACTTCGGAAACTATGCGGCTGCT | 174 |
| $S_5$ | AAGCAGCCGCATAGTAAAGGATTAAAAGCAATGGTCGTGG | 175 |
| Sequence set 5-8 numbering | Optimized sequence | |
| $S_1$ | AAGGCGAGGTCTTCAAAATGGTTGCTGGACGA | 176 |
| $S_2$ | ATCGTCCAGCAACCAAAAAATCCAAATGAGCGTGTTACGG | 177 |
| $S_3$ | ACCGTAACACGCTCAAAACCGAAGTGCCAATT | 178 |
| $S_4$ | AAATTGGCACTTCGGAAACTATGCGGCTGCT | 179 |
| $S_5$ | AAGCAGCCGCATAGTAAAGGATTAAATGAAGACCTCGCCT | 180 |
| Sequence set 5-9 numbering | Optimized sequence | |
| $S_1$ | ATCAAGGCGACCAGTAAAAGCTCCTCGACGA | 181 |
| $S_2$ | ATCGTCGAGGAGCTTAAAAATCCAAATGAGCGTGTTACGG | 182 |
| $S_3$ | ACCGTAACACGCTCAAAACCGAAGTGCCAATT | 183 |
| $S_4$ | AAATTGGCACTTCGGAAACTATGCGGCTGCT | 184 |
| $S_5$ | AAGCAGCCGCATAGTAAAGGATTAAAACTGGTCGCCTTGA | 185 |
| Sequence set 5-10 numbering | Optimized sequence | |
| $S_1$ | ATTCAGGCGACTCCTAAAGCACGACGATGGT | 186 |
| $S_2$ | AACCATCGTCGTGCTAAAAATCCAAATGAGCGTGTTACGG | 187 |
| $S_3$ | ACCGTAACACGCTCAAAACCGAAGTGCCAATT | 188 |

(continued)

| Sequence set 5-10 numbering | Optimized sequence | |
|---|---|---|
| $S_4$ | <u>A</u>AATTGGCACTTCGG<u>AAA</u>CTATGCGGCTGCT | 189 |
| $S_5$ | <u>A</u>AGCAGCCGCATAGT<u>AAA</u>GGATT<u>AAA</u>AGGAGTCGCCTGAA | 190 |
| Sequence set 5-11 numbering | Optimized sequence | |
| $S_1$ | <u>A</u>AGCACCTGCAATCC<u>AAA</u>TCGCCAGGACAAGT | 191 |
| $S_2$ | <u>A</u>ACTTGTCCTGGCGA<u>AAA</u>TGAGCAACCATGCC | 192 |
| $S_3$ | <u>A</u>GGCATGGTTGCTCA<u>AAA</u>CCGAACGTCGTGAT | 193 |
| $S_4$ | <u>A</u>ATCACGACGTTCGG<u>AAA</u>CTATGGAGCGGCT | 194 |
| $S_5$ | <u>A</u>AGCCGCTCCATAGT<u>AAA</u>GGATTGCAGGTGCT | 195 |
| Sequence set 5-12 numbering | Optimized sequence | |
| $S_1$ | <u>A</u>ACCTGCTGCAATCC<u>AAA</u>TCGCCACCTCAAGA | 196 |
| $S_2$ | <u>A</u>TCTTGAGGTGGCGA<u>AAA</u>TGAGCCTGGACGTT | 197 |
| $S_3$ | <u>A</u>AACGTCCAGGCTCA<u>AAA</u>CCGAACTGGTGCTT | 198 |
| $S_4$ | <u>A</u>AAGCACCAGTTCGG<u>AAA</u>CTATGCCGCTCCT | 199 |
| $S_5$ | <u>A</u>AGGAGCGGCATAGT<u>AAA</u>GGATTGCAGCAGGT | 200 |
| Sequence set 5-13 numbering | Optimized sequence | |
| $S_1$ | <u>A</u>AGCTGGTGCAATCC<u>AAA</u>TCGCCTCCTGACAA | 201 |
| $S_2$ | <u>A</u>TTGTCAGGAGGCGA<u>AAA</u>TGAGCAAGGTTGGC | 202 |
| $S_3$ | <u>A</u>GCCAACCTTGCTCA<u>AAA</u>CCGAACGCAGATGT | 203 |
| $S_4$ | <u>A</u>ACATCTGCGTTCGG<u>AAA</u>CTATGGAGCGGCA | 204 |

(continued)

| Sequence set 5-13 numbering | Optimized sequence | |
|---|---|---|
| $S_5$ | <u>A</u>TGCCGCTCCATAGT<u>AAA</u>GGATTGCACCAGCT | 205 |
| Sequence set 5-14 numbering | Optimized sequence | |
| $S_1$ | <u>A</u>TGCACGCACAATCC<u>AAA</u>TCGCCATCAGAGGT | 206 |
| $S_2$ | <u>A</u>ACCTCTGATGGCGA<u>AAA</u>TGAGCTGCCTCCAT | 207 |
| $S_3$ | <u>A</u>ATGGAGGCAGCTCA<u>AAA</u>CCGAACGTCGTCAT | 208 |
| $S_4$ | <u>A</u>ATGACGACGTTCGG<u>AAA</u>ACTATCGAGCGGCT | 209 |
| $S_5$ | <u>A</u>AGCCGCTCGATAGT<u>AAA</u>GGATTGTGCGTGCA | 210 |
| Sequence set 5-15 numbering | Optimized sequence | |
| $S_1$ | <u>A</u>AGCGTCGTGAATCC<u>AAA</u>TCGCCATCAGACCA | 211 |
| $S_2$ | <u>A</u>TGGTCTGATGGCGA<u>AAA</u>TGAGCAAGGCTCGT | 212 |
| $S_3$ | <u>A</u>ACGAGCCTTGCTCA<u>AAA</u>CCGAACCAGCTTGT | 213 |
| $S_4$ | <u>A</u>ACAAGCTGGTTCGG<u>AAA</u>ACTATGCGGCAGGT | 214 |
| $S_5$ | <u>A</u>ACCTGCCGCATAGT<u>AAA</u>GGATTCACGACGCT | 215 |
| Sequence set 5-16 numbering | Optimized sequence | |
| $S_1$ | <u>A</u>TCAGCACGCAATCC<u>AAA</u>TCGCCAGTTCAACC | 216 |
| $S_2$ | <u>A</u>GGTTGAACTGGCGA<u>AAA</u>TGAGCAAGCAGGCT | 217 |
| $S_3$ | <u>A</u>AGCCTGCTTGCTCA<u>AAA</u>CCGAACGTGGTGTT | 218 |
| $S_4$ | <u>A</u>AACACCACGTTCGG<u>AAA</u>ACTATGGAGCGGCA | 219 |
| $S_5$ | <u>A</u>TGCCGCTCCATAGT<u>AAA</u>GGATTGCGTGCTGA | 220 |

(continued)

| Sequence set 5-17 numbering | Optimized sequence | |
|---|---|---|
| $S_1$ | <u>A</u>AGCTGCACCAATCC<u>AAA</u>TCGCCAGAAGGTCA | 221 |
| $S_2$ | <u>A</u>TGACCTTCTGGCGA<u>AAA</u>TGAGCACGACGCAT | 222 |
| $S_3$ | <u>A</u>ATGCGTCGTGCTCA<u>AAA</u>CCGAACAACCTGCT | 223 |
| $S_4$ | <u>A</u>AGCAGGTTGTTCGG<u>AAA</u>CTATGGAGCGGCA | 224 |
| $S_5$ | <u>A</u>TGCCGCTCCATAGT<u>AAA</u>GGATTGGTGCAGCT | 225 |
| Sequence set 5-18 numbering | Optimized sequence | |
| $S_1$ | <u>A</u>ACGCTCGTCAATCC<u>AAA</u>TCGCCTCAGGACAA | 226 |
| $S_2$ | <u>A</u>TTGTCCTGAGGCGA<u>AAA</u>TGAGCCAACGACCT | 227 |
| $S_3$ | <u>A</u>AGGTCGTTGGCTCA<u>AAA</u>CCGAAGCTGGTGTT | 228 |
| $S_4$ | <u>A</u>AACACCAGCTTCGG<u>AAA</u>CTATGCCGCACCT | 229 |
| $S_5$ | <u>A</u>AGGTGCGGCATAGT<u>AAA</u>GGATTGACGAGCGT | 230 |
| Sequence set 5-19 numbering | Optimized sequence | |
| $S_1$ | <u>A</u>AGTGCGTCGAATCC<u>AAA</u>TCGCCAAGACCTCA | 231 |
| $S_2$ | <u>A</u>TGAGGTCTTGGCGA<u>AAA</u>TGAGCAGGCTGGAA | 232 |
| $S_3$ | <u>A</u>TTCCAGCCTGCTCA<u>AAA</u>CCGAAGCAACGTGT | 233 |
| $S_4$ | <u>A</u>ACACGTTGCTTCGG<u>AAA</u>CTATGCCGCTCCT | 234 |
| $S_5$ | <u>A</u>AGGAGCGGCATAGT<u>AAA</u>GGATTCGACGCACT | 235 |
| Sequence set 5-20 numbering | Optimized sequence | |
| $S_1$ | <u>A</u>TCACGCAGCAATCC<u>AAA</u>TCGCCATCACAACG | 236 |

(continued)

| Sequence set 5-20 numbering | Optimized sequence | |
|---|---|---|
| $S_2$ | ACGTTGTGATGGCGAAAATGAGCACGAGCCTT | 237 |
| $S_3$ | AAAGGCTCGTGCTCAAAACCGAAGGTTGCACT | 238 |
| $S_4$ | AAGTGCAACCTTCGGAAAACTATGCCGCTCCA | 239 |
| $S_5$ | ATGGAGCGGCATAGTAAAGGATTGCTGCGTGA | 240 |

In the seventh aspect of the present invention, it provides a device for determining the nucleic acid single strand sequence for forming the multimeric complex based on the complementary nucleic acid backbone, which comprises:

(M1) an input module, which is used to input annealing algorithm parameters, optimized constraint parameters, and optionally nucleic acid sequences to be optimized;
wherein the setting annealing algorithm parameters including: initial annealing temperature, annealing termination temperature, and annealing temperature attenuation coefficient $\Delta T$;
the optimized constraint parameters including:

① the number n of the nucleic acid single strand, preferably a positive integer of 3-6;
② the length $L$ of the pairing sequence, preferably the $L$ is of 12-16 bases;
③ the dissociation temperature threshold $T_m$ of the pairing region;
④ the free energy threshold $\Delta G_S^\circ$ of the specific pairing region sequence;
⑤ the free energy threshold $\Delta G_{NS}^\circ$ of the non-specific pairing;
⑥ the connecting element X2, preferably A, AA, and AAA;
⑦ the dissociation temperature threshold $T_{m-H}$ of the secondary structure (hairpin);
⑧ the CG proportion $P_{CG}$ in the pairing sequence, preferably the range of $P_{CG}$ is [0.4,0.6),,

(M2) an optimization operation module, which is configured to perform the following sub steps to obtain optimized nucleic acid single strand sequences or sets thereof:

(z1) calculating the objective function value $E_0$ of the initial set $S$, that is, calculating the sum of the non-specific pairing free energies ( $\Delta G_{NS}^\circ$ ) between sequences and of the sequence itself, while obtaining the non-specific pairing free energy matrix $C_{n \times n}$, searching the $S_i$ and $S_j$ ( $1 \le i \le n$, $1 \le j \le n$ ) corresponding to the minimum value in the upper triangular matrix thereof, randomly selecting $S_i$ or $S_j$ for an updated operation according to the non-specific pairing free energy of the $S_i$ and $S_j$ $\Delta G_{NS}^\circ(S_i, S_j)$ , and then obtaining a new nucleic acid sequence, thereby obtaining a updated sequence set $S'$;

(z2) determining whether the sequences in the set $S'$ of the previous step meet the set optimized constraint parameter conditions, verifying the following parameters, including the dissociation temperature $T_m$ of the specific pairing region, the free energy $\Delta G_S^\circ$ of the specific pairing region sequence, the dissociation temperature $T_{m-H}$ of the secondary structure and the CG proportion $P_{CG}$. If the above parameters meet the constraint conditions, the step (z3) is proceeded; otherwise, the step (z2) is repeated. If the step is performed 15 times continuously at a certain annealing temperature without obtaining the $S'$ that meets the conditions, then the set S becomes the set $S'$ and the next step is proceeded to prevent a dead cycle;

(z3) calculating the objective function value $E_1$ of the set $S'$ of the previous step, and comparing $E_0$ with $E_1$. If $E_1 \geq E_0$, it indicates that the non-specific pairing free energy can be optimized, and the sequence set $S'$ becomes the sequence set S. If $E_1 < E_0$, it indicates that the non-specific pairing free energy has not been optimized, and in this case, it is necessary to determine whether to accept the set $S'$ as $S$ according to the Metropolis criterion; and

(z4) the annealing temperature is attenuated according to the set attenuation coefficient $\Delta T$, and the steps (z1), (z2), and (z3) are repeated for the $S$ of the previous step, which is the Monte Carlo-based annealing algorithm, until the annealing temperature reaches the annealing termination temperature. The $S = \{S_1, S_2, ..., S_n\}$ of the previous step becomes the nucleic acid single strand sequence for forming the multimeric complex based on the complementary nucleic acid backbone; and

(M3) an output module, which is used to output optimized nucleic acid single strand sequences or sets thereof.

In another preferred embodiment, the optimized constraint parameters further comprises: for a tetramer (n=4), using a symmetric sequence to initialize a sequence set $S = \{S_1, S_2, ... , S_n\}$ according to the above parameters;

It should be understood that within the scope of the present invention, the above-mentioned technical features of the present invention and the technical features specifically described in the following (such as the embodiments) can be combined with each other to form a new or preferred technical solution, which are not redundantly repeated one by one due to space limitation.

**Description of drawings**

Figure 1 shows a schematic diagram of the multimer.

Figure 2 shows a flowchart of the annealing algorithm involved in the present patent.

Figure 3 shows a schematic diagram of specific sequence pairing of a trimer.

Figure 4 shows a gel electrophoresis diagram of the nucleic acid backbone assembly of the trimer optimized sequences.

Figure 5 shows a schematic diagram of specific sequence pairing of the tetramer optimized sequences.

Figure 6 shows a statistical diagram of the sum of free energies of non-specific pairing regions between the tetramer optimized sequences.

Figure 7 shows a gel electrophoresis diagram of the nucleic acid backbone assembly of the tetramer optimized sequences.

Figure 8 shows a schematic diagram of the conversion of a tetramer into a pentamer.

Figure 9 shows a statistical diagram of the sum of free energies in the unpaired regions of the optimized sequences for the first conversion scheme of pentamers.

Figure 10 shows a gel electrophoresis diagram of the nucleic acid backbone assembly of the optimized sequences for the first conversion scheme of pentamers.

Figure 11 shows a schematic diagram of specific sequence pairing of the optimized sequences for the second conversion scheme of pentamers.

Figure 12 shows a statistical diagram of the sum of free energies in the unpaired regions of the optimized sequences for the second conversion scheme of pentamers.

Figure 13 shows a gel electrophoresis diagram of the nucleic acid backbone assembly of the optimized sequences for the second conversion scheme of pentamers.

Figure 14 shows the coupling between G-CSF and L-DNA.

Figure 15 shows the purification effect of the (L-DNA)-G-CSF conjugate.

Figure 16 shows the assembly effect of the monovalent, divalent, and trivalent G-CSF complex.

Figure 17 shows the effect of the L-DNA tetramer framework on the in vitro activity of G-CSF.

Figure 18 shows the in vitro activity evaluation of the divalent and trivalent G-CSF assembled by L-DNA tetramer.

Figure 19 shows a. purification of SM(PEG)$_2$-PMO1 using HiTrap Capto MMC; b. gel electrophoresis diagram of coupling efficiency between SM(PEG)$_2$-PMO1 and anti-HSA nanoantibody.

Figure 20 shows the identification of PMO1(a), SM(PEG)$_2$-PMO1(b), anti-HSA Nb(c), and anti-HSA Nb-PMO1(d) by positive ion mode of liquid chromatography-mass spectrometry.

Figure 21 shows the separation of nanoantibodies and PMO nanoantibody conjugates using Superdex™ 75 Increase 10/300 GL.

Figure 22 shows a gel electrophoresis diagram and schematic diagram of the NAPPA-PMO assembly sample. Left: pmo-NAPPA4-HSA(1,2,3); Right: pmo-NAPPA4-HSA(1).

Figure 23 shows the binding activity of the anti-HSA Nb, anti-HSA Nb-PMOI, and pmo-NAPPA4-HSA(1) with human serum albumin protein detected by ELISA.

Figure 24 shows the resistance experiment of the pmo-NAPPA4-HSA(1) to nuclease degradation. Left: SDS-PAGE gel electrophoresis diagram of the pmo-NAPPA4-HSA(1) treated with three kinds of nuclease; Right: gel electrophoresis diagram of DDNA-NAPPA4 treated with three kinds of nuclease.

**Modes for carrying out the invention**

After extensive and intensive research, the inventors have developed a multivalent protein drug and its library, as well as a preparation method and application thereof for the first time. By using the drug library and preparation method of the present invention, short-acting protein drugs can be quickly, efficiently formed into multivalent complexes at low-cost, and with high yield, to increase the drug half-life, or form high valent antigens with monomer antigens to enhance their immunogenicity according to needs. On this basis, the inventors have completed the present invention.

Specifically, the present invention provides a multivalent protein drug, comprising n protein drug units, wherein each drug unit comprises a drug element moiety of the same kind and different nucleic acid element moieties connected to the drug element moiety; n is a positive integer≥2; n of different nucleic acid element moieties form n-multimers through nucleic acid base complementation, thereby forming the multivalent protein drug; a stable pairing structure with nucleic acid base complementation (rather than complex peptide bonds or other chemical modifications, etc.) of the multivalent protein drug of the present invention can be formed through rapid assembly (such as 1 minute). Experiments have shown that the molecular weight of the drug of the present invention can be increased through high-valent formation, thereby extending its half-life in animals.

In addition, based on the same implementation method, the drug element can also be an antigen used for vaccine development; the difference is that each antigen unit comprises the same or different antigen element moiety, as well as different nucleic acid element moieties connected to the antigen element moiety; n of different nucleic acid element moieties can form a n-multimer through nucleic acid base complementation, thereby forming the multivalent antigen.

Finally, the present invention provides a highly optimized nucleic acid sequence library, including nucleic acid sequence groups that can be efficiently and accurately assembled into 2-5 aggregates, for rapid and accurate self-assembly of the aforementioned drugs or antigen units into multivalent macromolecular complexes.

**Term**

Unless otherwise defined, all technical and scientific terms used herein have the same meanings as those commonly understood by common technicians in the art to which the present invention belongs. As used herein, when referring to specific listed values, the term "about" means that the values can vary by no more than 1% from the listed values. For example, as used herein, the expression of "about 100" includes all values between 99 and 101 (such as 99.1, 99.2, 99.3, 99.4, etc.).

**Drug D**

In the present invention, the drug element moiety comprises protein drugs and polypeptide drugs.

Typically, the protein drugs include but are not limited to cytokines, hormones (such as insulin, growth hormone, etc.), antibody drugs, and polypeptides.

In a preferred embodiment of the present invention, the protein drug is G-CSF for treating leukopenia.

**Antigen Library A**

The present invention provides an antigen library, which comprises N of antigen units;

wherein, the antigen units comprise an antigen element moiety and a nucleic acid element moiety connected to the antigen element moiety; different nucleic acid element moieties can form a n-multimer through nucleic acid base complementation, thereby forming the multivalent antigen;

wherein, the antigen element moiety is a protein antigen or a polypeptide antigen;

typically, the protein antigen or polypeptide antigen includes but is not limited to a virus, bacterial protein, structural region thereof, and fragment;

In the present invention, the antigen element moiety is partially selected from M of different antigen proteins in the library, M≤N; M of different antigen proteins contain different proteins in a certain virus or mutants of the same protein from different virus strains;

In a preferred embodiment of the present invention, the protein antigen is derived from novel coronavirus SARS-CoV-2; specifically, it is a high-valent antigen formed by the receptor binding domain (RBD) of the viral spike protein.

**Left-handed nucleic acid**

Left-handed nucleic acid refers to the mirror image of the natural right-handed nucleic acid (D-nucleic acid), which can be divided into left-handed DNA (L-DNA) and left-handed RNA (L-RNA). Left-handed (chiral center) mainly exists in the deoxyribose or ribose portion of nucleic acids, exhibiting mirror flipping. Therefore, Left-handed nucleic acid cannot be degraded by ubiquitous nuclease (such as exonuclease and endonuclease) in plasma.

**Preparation Method**

**1. Design and preparation of L-nucleic acid strand framework**

According to the present invention, an L-nucleic acid strand framework is formed by base complementation of two or more L-nucleic acid single strands. The 5' end or 3' end of each L-nucleic acid single strand is activated into groups (such as NH2, etc.) that can be subsequently modified, and then one end of the linker (such as SMCC, SBAP, etc.) is coupled with the activate group on the L-nucleic acid single strand. L-nucleic acids with the linkers can be assembled into the desired L-nucleic acid strand framework. In another preferred embodiment, the activated functional groups at the 5' or 3' end of the L-nucleic acid single strand (such as aldehide, maleimide, etc.) are already included in nucleic acid synthesis. After confirming that the L-nucleic acid with the linker can successfully self-assemble into a framework, the L-nucleic acid single strand with the linker can be separately coupled with antibodies for subsequent assembly. The L-nucleic acid framework of the present invention can be prepared by the following steps.

**1.1 Design of L-nucleic acid single strand for rapid self-assembly**

The required multivalent number n (such as a trimer, tetramer) is determined; the required number n of L-nucleic acid single strands based on the multivalent number n is determined; a corresponding number of L-nucleic acid single strand sequences is designed, and the stability of the target nucleic acid framework is regulated by optimizing base pairing, and the possibility of non-specific pairing between nucleic acid strands is reduced. The details of nucleic acid sequence design are specifically described in the summary of the invention and embodiments.

**1.2 Activation of L-DNA or L-RNA**

The activation of L-nucleic acid involves modification of its active group at the 5' end (X1) or 3' end (X3) and subsequent conjugation with the linkers. The modification of active groups can be customized by nucleic acid synthesis companies; The linkers generally have a bifunctional group, that is, one end can be coupled with an active nucleic acid group, and the other end can be connected to specific sites on the protein (such as NH3, SH).

According to a preferred embodiment of the present invention, all L-nucleic acids that make up the framework are modified with aldehydes at the 5' end, thereby completing the activation of L-nucleic acids and subsequently coupling to the N-terminal $\alpha$-amine of the protein.

**2. Preparation method of protein-L-nucleic acid complex**

First, the 5' or 3' end of L-nucleic acids is modified with aldehydes, and then, the aldehyde groups of L-nucleic acids are specifically connected to the N-terminal $NH_3$ of the protein through a reductive amination reaction under low pH (5-6) conditions.

**Algorithm and algorithm optimized nucleic acid sequences**

The present invention also provides a method and device of determining a nucleic acid single strand sequence for forming a multimeric complex based on a complementary nucleic acid backbone. Preferably, the method comprises a preferred algorithm of the present invention.

Typically, the nucleic acid sequence library optimized by computer algorithms of the present invention can include: (a) a nucleic acid sequence that can self-assemble into a trimer through base complementation; (b) a nucleic acid sequence that can self-assemble into a tetramer through base complementation; and (c) a nucleic acid sequence that can self-assemble into a pentamer through base complementation.

The complexes formed by representative nucleic acid sequences are trimer molecules, tetramer molecules, and pentamer molecules as shown in Figure 1.

Preferably, the nucleic acid sequence W of the present invention has the structure shown in formula 1:

$$W=X1–R1–X2–R2–X3 \qquad (1)$$

wherein,

R1 is the complementary base pairing region 1;
R2 is the complementary base pairing region 2;
X1, X2, and X3 are none or redundant nucleic acids, independently;
the length of R1 and R2 is 14-16 bases;
the length of X1 and X3 is 0-5 bases;
X2 has a length of 0-3 bases and a sequence of A, AA, AGA or AAA;
wherein, the R1 of the nucleic acid sequence forms a target pairing with the R2 of different nucleic acid sequences, while the R2 of the nucleic acid sequence forms a target pairing with the R1 of another nucleic acid sequence.

In the present invention, the self-pairing of any region of the nucleic acid sequence belongs to non-target pairing, which needs to be avoided in design.

Preferably, the nucleic acid sequence of the present invention can be designed or optimized using a computer algorithm of Simulated Annealing (SA).

The computer algorithm minimizes the free energy ($\Delta G^{\circ}$) of the DNA double strand structure formed by target pairing, while maximizing the $\Delta G^{\circ}$ of non-target pairing;

specifically, the stability of DNA double strand structure depends on the base pairs of each nearest neighbor in the sequence; there may be 10 different nearest neighbor interactions in any Watson-Crick DNA double strand structure, and these pairing interactions are: AA/TT; AT/TA; TA/AT; CA/GT; GT/CA; CT/GA; GA/CT; CG/GC; GC/CG; and GG/CC;

more specifically, the $\Delta G^{\circ}$ values of the 10 base pairs mentioned above can be calculated by enthalpy ($\Delta H^{\circ}$) and entropy ($\Delta S^{\circ}$) at any temperature; enthalpy, entropy, and free energy data for 10 sequences summarized in Table 1.

Table 1 Thermodynamic data for 10 sequences

| Sequence | $\Delta H^{\circ}$ kcal/mol | $\Delta S^{\circ}$ cal K$^{-1}$ mol$^{-1}$ | $\Delta G_2^{\circ}$ kcal/mol | kcal/mol |
|---|---|---|---|---|
| AA/TT | -9.1 | -24.0 | -1.9 | -1.94 |
| AT/TA | -8.6 | -23.9 | -1.5 | -1.47 |
| TA/AT | -6.0 | -16.9 | -0.9 | -0.96 |
| CA/GT | -5.8 | -12.9 | -1.9 | -1.95 |
| GT/CA | -6.5 | -17.3 | -1.3 | -1.34 |
| CT/GA | -7.8 | -20.8 | -1.6 | -1.6 |
| GA/CT | -5.6 | -13.5 | -1.6 | -1.57 |
| CG/GC | -11.9 | -27.8 | -3.6 | -3.61 |
| GC/CG | -11.1 | -26.7 | -3.1 | -3.14 |
| GG/CC | -11.0 | -26.6 | -3.1 | -3.07 |

Note: $\Delta H^{\circ}$, $\Delta S^{\circ}$ and $\Delta G_1^{\circ}$ are measured under conditions of 1 M NaCl, 25°C, and pH 7. $\Delta G_2^{\circ}$ is measured from the IDT website (https://sg.idtdna.com/calc/analyzer).

Using the thermodynamic values in Table 1, the enthalpy $\Delta H^{\circ}$ and free energy $\Delta G^{\circ}$ values of DNA oligomers can be effectively predicted by the nearest neighbor method. Taking the complementary pairing of GGAATTCC/CCTTAAGG as an example, using the nearest neighbor method, it is calculated to be $\Delta G^{\circ}$= -14.63 kcal/mol.

The annealing algorithm not only optimizes the $\Delta G_{NS}^{\circ}$ values, but also implements constraints on the dissociation temperature of nucleic acid sequence pairing ($T_m$) to ensure $T_m > 50$°C (when $L = 14$ bases) of R1 and R2 regions.

The nearest neighbor model is based on thermodynamic calculations and accurately predicts the stability of DNA double strands. The prediction of a given base sequence is provided by the model based on the nearest neighbor base pairs. The calculation of the unwinding temperature involves enthalpy ($\Delta H^{\circ}$) and entropy ($\Delta S^{\circ}$), and the calculation method is as follows:

$$T_m = \frac{\Delta H^{\circ}}{\Delta S^{\circ}[Na^+] + R \times \ln(\text{CT})} - 273.15 \qquad (1)$$

wherein, the R is a constant (1.987 cal K$^{-1}$mol$^{-1}$), the CT is the strand concentration given as 0.1 $\mu$M, the $\Delta S^{\circ}[Na^+]$ is the entropy value of the DNA double strand at a given sodium ion concentration, and the $\Delta H^{\circ}$ is the enthalpy value under given conditions.

Simulated annealing is a universal probability algorithm, and it is a method for approximate optimal solution of the problem, which is designed according to Monte Carlo's ideas, aiming to find the approximate optimal solution in a large searching space within a certain period of time. The idea of simulated annealing algorithm originates from the annealing process of solid materials in physics: first, the solid is fully heated, and then slowly cooled. When heated, the internal energy of free motion of particles inside the solid increases. Later, as the temperature gradually decreases, the particles tend to become orderly and reach equilibrium at each temperature. If the temperature drops slowly enough near the condensation point, the ground state can be reached, and the internal energy is minimized. According to the Metropolis criterion, the probability of a particle reaching equilibrium at temperature $T$ is exp(-$\Delta E/(KT)$), wherein the E is the internal energy at temperature $T$, the $\Delta E$ is its variation, and the $K$ is the Boltzmann constant. For the combinatorial optimization problem, there is a similar process. The solid micro state i is simulated as a solution X, the objective function is equivalent to the internal energy $E_i$ of state $i$, and the solid temperature is simulated with the control parameter T. The iteration process of "generating a new solution → calculating the objective function difference → judging whether to accept → accepting/discarding" for each value of T is repeated, and the T value is gradually attenuated. During the iteration process, a poor solution is limitedly accepted according to the Metropolis criterion, and the probability of accepting the poor solution is gradually approaching zero, so as to find the global optimal solution at all possible when the algorithm terminates.

In the present invention, the free energy of the non-target pairing region between the single strands of a multimeric nucleic acid is simulated as internal energy. While ensuring the free energy and dissociation temperature of the target pairing region between the nucleic acid single strands, the free energy of the non-target pairing region is optimized through iteration of a simulated annealing algorithm. Finally, the optimized single strand is more conducive to the assembly of the multimer. The optimized objective function used herein is as follows:

$$E = \sum_{i=1}^{n} \sum_{j=i}^{n} -\Delta G^{\circ}(S_i, \ S_j), n \geq 1 \qquad (2)$$

$\Delta G^{\circ}(S_i, S_j)$ is the free energy of the non-target pairing between $S_i$ sequence and $S_j$ sequence, and $\sum_{i=1}^{n} \sum_{j=i}^{n} \Delta G^{\circ}(S_i, \ S_j)$ is the sum of the free energies of non-target pairing between all sequences, with a negative value; wherein, the larger the negative value, the more beneficial it is to reduce the non-target pairing. Therefore, an objective function is constructed based on the idea of minimizing energy using a degradation algorithm, and a minus sign is added before $\Delta G^{\circ}(S_i, S_j$, converting it into a positive number. At this point, the smaller the value of $\sum_{i=1}^{n} \sum_{j=i}^{n} -\Delta G^{\circ}(S_i, \ S_j)$, the more beneficial it is to reduce non-target pairing.

The flowchart of a representative algorithm of the present invention is shown in Figure 2.

In the present invention, the simulated annealing algorithm introduces random factors, and in each iteration update process, it will accept a solution that is worse than the current one with a certain probability, so it is possible to jump out of the local optimal solution and reach the global optimal solution.

**Multivalent macromolecular complexes**

The present invention also provides a multivalent macromolecular complex with improved drug half-life and activity, which is formed by using the nucleic acid multimer to mediate protein drugs, wherein the nucleic acid multimer is designed using the above algorithm.

Preferably, the nucleic acid sequence is a nucleic acid sequence set which can be specifically assembled into n-multimers in a nucleic acid sequence library;

preferably, in the present invention, the protein drug is a protein drug that needs multivalent formation to increase its half-life or activity.

Typically, each nucleic acid strand of the nucleic acid sequence set is connected to the protein drug, forming a protein drug-nucleic acid strand unit, with the structure shown in formula 2:

$$D-[L-W_i], \quad i=1 \text{ to n} \qquad (2)$$

wherein,

D is the protein drug element moiety;

each $W_i$ is independently a nucleic acid sequence; the nucleic acid sequence is selected from the group consisting of: left-handed nucleic acid, peptide nucleic acid, locked nucleic acid, thio-modified nucleic acid, 2'-fluoro modified nucleic acid, 5-hydroxymethylcytosine nucleic acid, and combinations thereof; the nucleic acid sequence has the structure shown in formula 1, and is selected from the nucleic acid sequence set that can form n-multimers mentioned above;

L is a linker; the linker moiety has already been included in the synthesis or preparation of $W_i$, connected to the X1 or X3 of $W_i$ (see formula 1);

"-" is a covalent bond;

In another preferred embodiment, the drug element moiety is selected from the group consisting of: protein drugs and polypeptide drugs that need to increase their molecular weights, thereby increasing their half-lives, and protein drugs and peptide drugs that need multivalent formation to increase their activities;

In another preferred embodiment, the L has aldehyde, NHS ester, or similar functional groups near the D-end, for connecting the N-terminal $\alpha$-amine or lysine $\varepsilon$-amine on D;

In another preferred embodiment, the L has a maleimide functional group or haloacetyl (such as bromoacetyl, iodoacetyl, etc.) functional group near the D end, for connecting the free thiol (-SH) functional group on D;

In another preferred embodiment, the D is selected from the group consisting of: natural proteins, recombinant proteins, chemically modified proteins, and synthesized polypeptides;

In another preferred embodiment, the D can have a site-directed modification or site-directed addition of non-natural amino acids for connecting the $L-W_i$ of formula 1;

the protein drugs are respectively connected to different L-W; that can be assembled into n-multimers, forming protein drug self-assembly units, $D-[L-W_1]$, $D-[L-W_2]$, ..., $D-[L-W_n]$;

the protein drug self-assembly units, $D-[L-W_1]$, $D-[L-W_2]$, ..., $D-[L-W_n]$, are mixed in equimolar solution to assemble multivalent molecular complexes of protein drugs.

The present invention also provides a multivalent macromolecular complex which enhances the effectiveness of vaccines in inducing neutralizing antibody production in vivo, and it is formed by using the nucleic acid multimer to mediate one or more antigens, wherein the nucleic acid multimer is designed using the above algorithm;

wherein, the nucleic acid sequence is a nucleic acid sequence set which can be specifically assembled into n-multimers in a nucleic acid sequence library;

wherein, the antigen is an antigen or antigen library; the antigen library comprises M of different antigen proteins, $1 \leq M \leq n$;

each nucleic acid strand of the nucleic acid sequence set is connected to an antigen in the antigen library, forming an antigen-nucleic acid strand unit with the structure shown in formula 3:

$$A_k-[L-W_i], \quad i=1\text{-}n, \quad k=1\text{-}M \qquad (3)$$

wherein,

$A_k$ is the antigen k in the antigen library; one $A_k$ corresponds to one or more $L-W_i$ (such as $A_1-[L-W_1]$, $A_1-[L-W_2]$, $A_2-[L-W_3]$, $A_3-[L-W_4]$);

the other aspects of formula 3 are the same as those of the above formula 3;

the antigen proteins are respectively connected to different $L-W_i$ that can be assembled into n-multimers, forming antigen self-assembly units, such as $A_1-[L-Wi]$, $A_2-[L-W_2]$, ..., $A_3-[L-W_N]$;

the antigen self-assembly units are mixed in equimolar solution to assemble multivalent antigen complexes.

**Main advantages of the present invention are:**

(1) the present invention can achieve multivalent formation of ready-made short-acting protein drugs without the need of the reconstruction of fusion proteins or complex chemical modification and cross-linking conditions, thereby improving their half-lives and activities; aldehyde modification of L-nucleic acids can specifically connect the N-terminal amine of proteins, forming protein drug units that can self-assemble into oligomers;
(2) the protein drug units (protein-nucleic acid connecting products) of the present invention can achieve multivalent formation of protein drugs within one minute through the mediation of a left-handed nucleic acid strand;
(3) in terms of vaccine development, the present invention can achieve multivalent formation of monomeric protein antigens, improving their immunogenicity;
(4) in terms of vaccine development, the present invention can also achieve the assembly of antigen mutations and subtypes of different virus or bacterial strains into diverse high-valent antigens, inducing a wider range of neutralizing antibodies.

The present invention will be further illustrated below with reference to the specific examples. It should be understood that these examples are only to illustrate the invention, not to limit the scope of the invention. The experimental methods with no specific conditions described in the following examples are generally performed under the conventional conditions (e.g., the conditions described by Sambrook et al., Molecular Cloning: A Laboratory Manual (New York: Cold Spring Harbor Laboratory Press, 1989), or according to the manufacturer's instructions. Unless indicated otherwise, percentages and portions are weight percentages and weight portions.

**Example 1: Assembly design, synthesis, and validation of a trimer nucleic acid backbone**

Three nucleic acids that can be paired according to the shape shown in Figure 1(A) are designed. Specifically, the specific complementary pairing of nucleic acid single strands $R_1$ with nucleic acid single strands $R_6$, instead of other nucleic acid single strands, is performed. Similarly, the specific complementary pairing of $R_3$, $R_5$ with $R_2$, $R_4$ respectively, instead of other nucleic acid single strands, is performed. And the free energy of specific complementary pairing ( $\Delta G_S^\circ$ ) is much smaller than that of non-specific pairing ( $\Delta G_{NS}^\circ$ ). The free energy of specific complementary pairing ( $\Delta G_S^\circ$ ) is less than - 29 kcal/mol, while the free energy of non-specific pairing ( $\Delta G_{NS}^\circ$ ) is greater than -7 kcal/mol. In this way, the form of trimers is the most stable in the reaction system. The specific implementation steps of trimer optimization are described in Figure 2; the annealing parameters are: annealing initial temperature $T_o$ = 50°C, annealing termination temperature $T_f$ = 0.12°C, annealing temperature decay coefficient $\Delta T$=0.98 (each attenuation of annealing initial temperature is 0.98 of the current value); the optimized constraint parameters are: pairing sequence length L=16 bases, dissociation temperature threshold $T_m$ > 54°C, pairing sequence free energy threshold: $\Delta G_S^\circ < -29$ kcal/mol, and non-specific pairing free energy threshold: $\Delta G_{NS}^\circ > -7$ kcal/mol. The specific implementation steps for optimization are as follows:

Sequence initialization: pairing sequences $R = \{R_1, R_3, R_5\}$ are initialized based on parameters, and $R_6$, $R_2$ and $R_4$ are obtained according to base complementation. After splicing the six sequences as shown in Figure 1 (A), the initial sequence set $S = \{S_1, S_2, S_3\}$ is finally obtained.

Generation of new solution: new solutions $S'$ are obtained by updating the sequence set $S$. First, $\|-\Delta G^\circ(S_i, S_j)\|_\infty$ is calculated to identify the two sequences $S_i$ and $S_j$ of non-specific pairings that have the greatest impact on the target pairing in the set S. Then, $S_i$ or $S_j$ in non-target pairing regions is randomly selected for update according to the $\Delta G_{NS}^\circ(S_i, S_j)$, thereby obtaining a new nucleic acid sequence. The dissociation temperature of the pairing regions ($T_m$) of this nucleic acid sequence is tested to see if it is greater than 54°C, and the free energy of the pairing regions ( $\Delta G_S^\circ$ ) is tested at the same time to see if it is less than -29 kcal/mol. If the constraint requirements of dissociation temperature and pairing region free energy ( $\Delta G_S^\circ$ ) are not met, the update will be repeated. If the constraint requirements of dissociation temperature and pairing region free energy ( $\Delta G_S^{\circ}$ ) are met, the $S$ is updated according to the principle of base complementation, and finally obtaining a new sequence set $S'$. If the new nucleic acid sequence obtained after fifteen updates still does not meet the constraints of dissociation temperature and pairing region free energy ( $\Delta G_S^{\circ}$ ), in order to prevent a dead cycle, the set S becomes the new solution $S'$.

Optimization judgment: the objective function values $E_i$ and $E_{i+1}$ of the set $S$ and $S'$ are calculated respectively according to formula 2. If $E_{i+1} - E_i \geq 0$, it indicates that the update has optimized the non-target pairing free energy ( $\Delta G_{NS}^{\circ}$ ), then $S \leftarrow S'$, the new solution $S'$ becomes $S$. If $E_{i+1} - E_i < 0$, it indicates that the update has obtained a deteriorating solution. According to the Metropolis criterion, the probability $p = \exp(-\Delta E/T_o)$ is calculated and r is randomly generated, $r \in [0,1)$. If $p > r$, then accept the deteriorating solution, otherwise reject the deteriorating solution. Finally, the annealing initial temperature decays to 0.98 of the current value, generating the next new solution until it decays to the annealing termination temperature, thereby obtaining an optimized sequence set S.

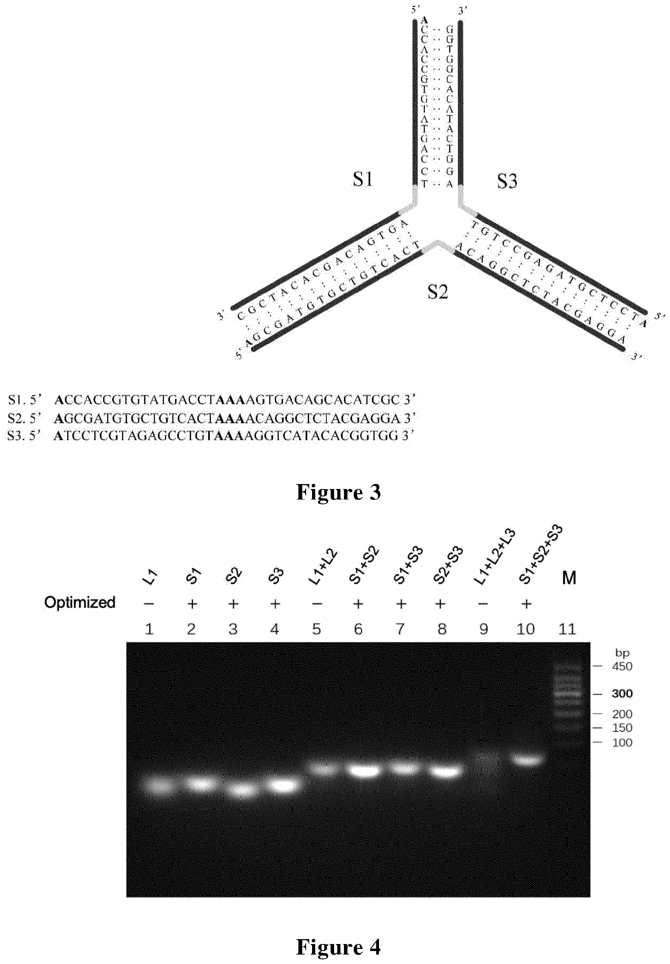

The optimized sequences in Table 2_1 are obtained through the above algorithm optimization. The purpose of the 5' end A of each sequence is to modify the active group for the subsequent coupling with linkers. In the non-target pairing free energy ( $\Delta G_{NS}^{\circ}$ ) matrix table and the target pairing region parameter index table, some main parameter values are counted. The schematic diagram of specific sequence pairing of the trimer optimized sequences is shown in Figure 3. From the corresponding gel electrophoresis diagram of the nucleic acid backbone (Figure 4), it can be seen that Lane9 is an artificially designed trimer with an unclear and trailing band. Lane 10 is the main band formed by the optimized sequences $S_1$, $S_2$ and $S_3$, indicating the formation of a trimer and exhibiting high stability.

Table2_1 Trimer initialized sequences and optimized sequences

| Sequence numbering | Initialized sequences | SEQ ID NO: |
|---|---|---|
| $S_1$ | AGTGATCCGAAGTCGAC<u>AAA</u>CGTATTAGCGCTCGAT | <u>241</u> |
| $S_2$ | <u>A</u>ATCGAGCGCTAATACG<u>AAA</u>GTGCAATGCGTCGATG | <u>242</u> |
| $S_3$ | <u>A</u>CATCGACGCATTGCAC<u>AAA</u>GTCGACTTCGGATCAC | <u>243</u> |
| Sequence numbering | Optimized sequences | |
| $S_1$ | <u>A</u>CCACCGTGTATGACCT<u>AAA</u>AGTGACAGCACATCGC | <u>244</u> |
| $S_2$ | <u>A</u>GCGATGTGCTGTCACT<u>AAA</u>ACAGGCTCTACGAGGA | <u>245</u> |
| $S_3$ | <u>A</u>TCCTCGTAGAGCCTGT<u>AAA</u>AGGTCATACACGGTGG | <u>246</u> |

Table 2_2 Trimer initialized sequence and optimized sequence free energy ( $\Delta G_{NS}^{\circ}$ ) matrix

| Trimer initialized free energy matrix | | | | Trimer optimized sequence free energy matrix | | | |
|---|---|---|---|---|---|---|---|
| Sequence | $S_1$ | $S_2$ | $S_3$ | Sequence | $S_1$ | $S_2$ | $S_3$ |
| $S_1$ | -13.09 | -8.1 | -8.24 | $S_1$ | -3.61 | -5.19 | -4.89 |
| $S_2$ | | -13.09 | -8.1 | $S_2$ | | -4.89 | -5.19 |
| $S_3$ | | | -16.53 | $S_3$ | | | -4.95 |

Table 2_3 Parameter indicators for target pairing regions of trimer optimized sequences

| Pairing sequences | CG% | $\Delta G_S^\circ$ | Tm °C (TF) | Tm °C (IDT) |
|---|---|---|---|---|
| $R_1$ | 56.3% | -29.66 | 55.3 | 50.9 |
| $R_3$ | 56.3% | -29.55 | 57.1 | 52.5 |
| $R_5$ | 56.3% | -29.61 | 54.4 | 51.6 |

**Example 2: Assembly design, synthesis, and validation of a tetramer nucleic acid backbone**

Four nucleic acids that can be paired according to the shape shown in Figure 1(B) are designed, wherein, the specific complementary pairing of nucleic acid single strands $R_1$, $R_3$, $R_5$ and $R_7$ with nucleic acid single strands $R_8$, $R_2$, $R_4$ , and $R_6$ respectively, instead of other nucleic acid single strands, is performed. And the free energy of specific complementary pairing ( $\Delta G_S^\circ$ ) is much smaller than that of non-specific pairing ( $\Delta G_{NS}^\circ$ ). The free energy of specific complementary pairing ( $\Delta G_S^\circ$ ) is less than -27.4 kcal/mol, while the free energy of non-specific pairing ( $\Delta G_{NS}^\circ$ ) is greater than -7 kcal/mol. In this way, the form of tetramers is the most stable in the reaction system. The specific implementation steps of tetramer optimization are described in Figure 2; the annealing parameters are: annealing initial temperature $T_o$ = 50°C, annealing termination temperature $T_f$ = 0.12°C, annealing temperature decay coefficient $\Delta T$=0.98 (each attenuation of annealing initial temperature is 0.98 of the current value); the optimized constraint parameters are: pairing sequence length L=14 bases, dissociation temperature threshold $T_m$ > 52°C, pairing sequence free energy threshold: $\Delta G_S^\circ < -27.4$ kcal/mol, and non-specific pairing free energy threshold: $\Delta G_{NS}^\circ > -7$ kcal/mol.

The specific implementation steps for optimization are as follows:

Sequence initialization: pairing sequences $R = \{R_1,R_3,R_5,R_7\}$ are initialized based on parameters, and $R_8$ , $R_2$ , $R_4$ , and $R_6$ are obtained according to base complementation. After splicing the eight sequences as shown in Figure 1 (B), the initial sequence set $S = \{S_1,S_2,S_3,S_4\}$ is finally obtained. During the tetramer experiment, it is found that a fixed core structure can effectively improve the assembly efficiency of nucleic acid sequences. Based on the nucleic acid sequence of formula 1, the nucleic acid sequence W2 is developed, which has the structure of formula 4:

$$W2 = X1–Q1–C1–X2–C2–Q2–X3 \qquad (4)$$

wherein, C1 and C2 are fixed core structure parts, and Q1 and Q2 are sequences other than fixed core structure.

Table 3 Nucleic acid sequences $S_1$, $S_2$, $S_3$ and $S_4$ containing fixed core structures

| $S_1$ | X1-Q1-AATCC-X2-TGAGC-Q2-X3 |
|---|---|
| $S_2$ | X1-Q3-GCTCA-X2-CCGAA-Q4-X3 |
| $S_3$ | X1-Q5-TTCGG-X2-ACTAT-Q6-X3 |
| $S_4$ | X1-Q7-ATAGT-X2-GGATT-Q8-X3 |

Generation of new solution: the same as the generation of new solution in Example 1. The difference is that if a fixed core structure is used, the update will not include the fixed core structure part; constraint parameters need to be strictly followed, the dissociation temperature ($T_m$) of the pairing regions of the new nucleic acid sequence should be greater than 52°C, and the free energy of the pairing regions ( $\Delta G_S^{\circ}$ ) should be less than -27.4 kcal/mol.

Optimization judgment: the same as the optimization judgment in Example 1.

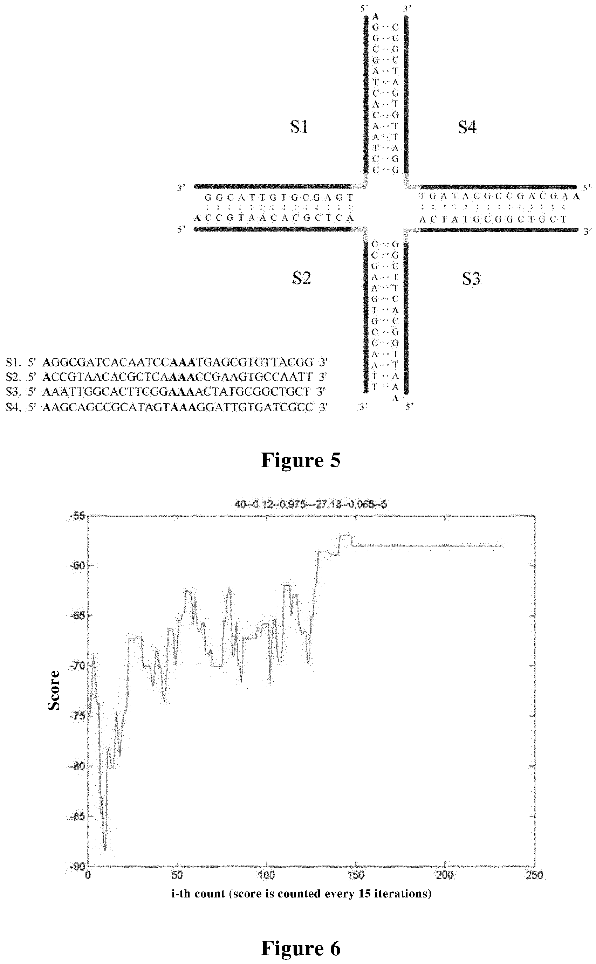

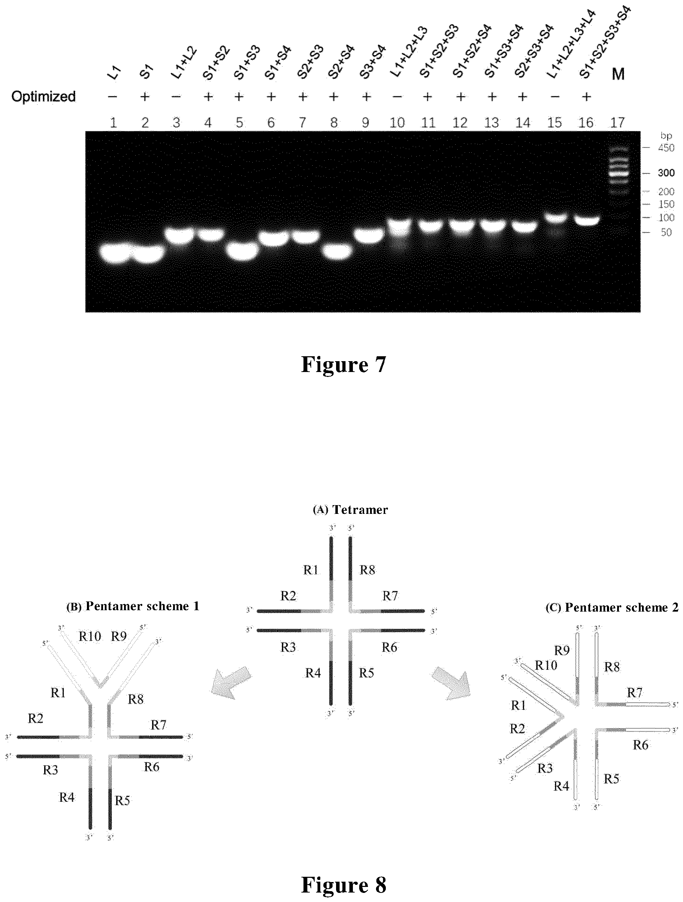

The optimized sequences in Table 4_1 are obtained through the above algorithm optimization, and the specific pairing diagram thereof is shown in Figure 5. The statistical line graph of the non-target pairing free energy during the optimization process of the tetramer optimized sequences is shown in Figure 6. In the case of adding connecting elements, the present invention seeks to avoid the significant impact on the free energy ( $\Delta G_{NS}^{\circ}$ ) of the optimized sequences which are not added with the connecting elements. Regardless of whether the connecting elements are added, the sum of the free energy ( $\Delta G_{NS}^{\circ}$ ) matrices of the optimized sequences should be as large as possible. Therefore, during the optimization process, the objective function values of the optimized sequences without the addition of the connecting elements are also counted, and the final detection is only performed on the optimized sequences with the addition of the connecting elements. From the corresponding gel electrophoresis diagram of the nucleic acid backbone (Figure 7), it can be seen that Lane15 is an artificially designed tetramer with a trailing band. Lane 16 is the main band formed by the optimized sequences $S_1$, $S_2$, $S_3$ and $S_4$, which is around 100 bp, indicating the formation of a tetramer and exhibiting high stability.

The above implementing steps for tetramers mainly optimize the non-specific pairing free energy between sequences, and the secondary structure formed by the self-folding of sequences also has a significant impact on the assembly of tetramers. If the dissociation temperature of the secondary structure formed by the sequence itself is too high, once this stable secondary structure is formed, it is difficult to break this state, making it difficult for the tetramer to assemble. Therefore, it is necessary to control the dissociation temperature of the secondary structures corresponding to the four sequences of the assembled tetramer to be not too high. For tetramers, it is necessary to control the dissociation temperature of the secondary structures of the four nucleic acid sequences. If a symmetric structure is used (as shown in Figure 1, $R_1$,$R_3$,$R_5$,$R_7$ maintain symmetry with $R_4$,$R_6$,$R_8$,$R_2$ , respectively), similar secondary structures will appear between the two sequences, and the dissociation temperature of the secondary structure between the two sequences is not significantly different. At this point, only the secondary structure of the two nucleic acid sequences needs to be controlled to achieve the previous effect. Therefore, symmetry is beneficial for controlling the secondary structure in tetramer optimization.

Table4_1 Tetramer initialized sequences and optimized sequences

| Sequence numbering | Initialized sequences | SEQ ID NO: |
|---|---|---|
| $S_1$ | AACCTGGTACAATCC<u>AAA</u>TGAGCTACACTAGC | <u>247</u> |
| $S_2$ | AGCTAGTGTAGCTCA<u>AAA</u>CCGAAGTATCGATT | <u>248</u> |
| $S_3$ | AAATCGATACTTCGG<u>AAA</u>ACTATAGTGAGTTG | 249 |
| $S_4$ | A<u>CAACTCACTATAGT</u>AAAGGATTGTACCAGGT | <u>250</u> |
| Sequence numbering | Optimized sequences | |
| $S_1$ | AGGCGATCACAATCC<u>AAA</u>TGAGCGTGTTACGG | <u>251</u> |
| $S_2$ | ACCGTAACACGCTCA<u>AAA</u>CCGAAGTGCCAATT | 252 |

(continued)

| Sequence numbering | Optimized sequences | |
|---|---|---|
| $S_3$ | AAATTGGCACTTCGGAAAACTATGCGGCTGCT | 253 |
| $S_4$ | AAGCAGCCGCATAGTAAAGGATTGTGATCGCC | 254 |

Table 4_2 Tetramer initialized sequence and optimized sequence free energy ( $\Delta G_{NS}^{\circ}$ ) matrix

| Tetramer initialized free energy matrix | | | | | Tetramer optimized sequence free energy matrix | | | | |
|---|---|---|---|---|---|---|---|---|---|
| Sequence | $S_1$ | $S_2$ | $S_3$ | $S_4$ | Sequence | $S_1$ | $S_2$ | $S_3$ | $S_4$ |
| $S_1$ | -6.34 | -6.34 | -5.85 | -5.13 | $S_1$ | -4.95 | -6.75 | -6.97 | -6.75 |
| $S_2$ | | -9.69 | -5.19 | -5.85 | $S_2$ | | -5.36 | -6.75 | -5.37 |
| $S_3$ | | | -9.69 | -4.99 | $S_3$ | | | -5.36 | -6.75 |
| $S_4$ | | | | -9.27 | $S_4$ | | | | -4.62 |

Table4_3 Parameter indicators for pairing regions of tetramer optimized sequences

| Pairing sequences | CG% | $\Delta G_S^{\circ}$ | Tm °C (TF) | Tm °C (IDT) |
|---|---|---|---|---|
| $R_1$ | 57.1% | -27.76 | 53.8 | 46.2 |
| $R_3$ | 57.1% | -27.44 | 53.0 | 48.0 |
| $R_5$ | 50.0% | -28.62 | 53.7 | 45.4 |
| $R_7$ | 57.1% | -28.58 | 53.6 | 50.2 |

**Example 3: Assembly design, synthesis, and validation of a pentamer nucleic acid backbone**

The tetramer optimized sequences exhibit excellent assembly performance, largely due to the lack of pairing in the central region of the tetramer, which means that the fixed core structure provides sufficient freedom for the tetramer and does not form complicated complexes in the central region. Therefore, in order to integrate the tetramer sequences and the fixed core structure in the optimization of the pentamer sequences, two schemes of utilizing the tetramer sequences and the fixed core structure in Example 2 are explored and designed.

The first conversion scheme: five nucleic acids that can be paired according to the shape shown in Figure 8(B) are designed. This scheme preserves the partial sequence and complete fixed core structure of the tetramer in Example 2, and the core structure is not opened. Open up the tetramer at $R_1$ and $R_8$ of the original tetramer, except for the core structure, and add two nucleic acid sequences $R_9$ and $R_{10}$ with a length of 14. Among them, nucleic acid single strands $R_1$, $R_3$, $R_5$, $R_7$ and $R_9$ are specifically complementary paired with nucleic acid single strands $R_{10}$, $R_2$, $R_4$, $R_6$ and $R_8$, respectively, without pairing with other nucleic acid single strands. The free energy of specific complementary pairing ( $\Delta G_S^{\circ}$ ) is less than -27.4 kcal/mol, while the free energy of non-specific pairing ( $\Delta G_{NS}^{\circ}$ ) is greater than -7 kcal/mol. In this way, the form of pentamers is the most stable in the reaction system. The specific implementation steps for optimizing the first conversion scheme of pentamers are described in Figure 2; the annealing parameters are: annealing initial temperature $T_o$ = 50°C, annealing termination temperature $T_f$ = 0.12°C, and annealing temperature decay coefficient $\Delta T$=0.9 (each attenuation of annealing initial temperature is 0.9 of the current value. Due to the use of partial sequences of tetramers, there are fewer updated regions and faster annealing temperature decay); the optimized constraint parameters are: pairing sequence length $L$=14 bases, dissociation temperature threshold $T_m$ > 52°C, pairing sequence

free energy threshold: $\Delta G_S^\circ < -27.4$ kcal/mol, and non-specific pairing free energy threshold: $\Delta G_{NS}^\circ > -7$ kcal/mol.

In this scheme, a complete tetramer fixed core structure is used, and the nucleic acid sequence W5 is developed based on the nucleic acid sequence of formula 4, while the nucleic acid sequence W6 is developed based on the nucleic acid sequence of formula 4. W5 has the structure of formula 7, and W6 has the structure of formula 8:

$$W3=X1–R1–X2–C1–X2–C2–Q1–X3 \qquad (5)$$

$$W4=X1–Q1–C1–X2–C2–X2–R1–X3 \qquad (6)$$

This scheme includes sequences of four structures: formula 1, formula 4, formula 5, and formula 6.

Table 5 Nucleic acid sequences $S_1$, $S_2$, $S_3$ , $S_4$ and $S_5$ containing partial core structures

| | |
|---|---|
| $S_1$ | X1-R9-X2-R10-X3 |
| $S_2$ | X1-R1-X2-AATCC-X2-TGAGC-Q1-X3 |
| $S_3$ | X1-Q2-GCTCA-X2-CCGAA-Q3-X3 |
| $S_4$ | X1-Q4-TTCGG-X2-ACTAT-Q5-X3 |
| $S_5$ | X1-Q6-ATAGT-X2-GGATT-X2-R8-X3 |

The specific implementation steps for optimization are as follows:
pairing sequences $R = \{R_9,R_{10}\}$ are initialized based on parameters, and $R_8$ and $R_1$ are obtained according to base complementation, $R_2$, $R_3$, $R_4$, $R_5$, $R_6$ and $R_7$ are from the homonymous optimized tetramer sequences in Example 2. $S_1$ is obtained by concatenating $R_9$ with $R_{10}$, $S_2$ is obtained by concatenating $R_1$ with $R_2$ according to formula 5, $S_3$ is obtained by concatenating $R_3$ with $R_4$, $S_4$ is obtained by concatenating $R_5$ with $R_6$, $S_5$ is obtained by concatenating $R_7$ with $R_8$ according to formula 6, and the initialized sequence set $S = \{S_1,S_2,S_3,S_4,S_5\}$ is finally obtained.

Generation of new solution: Randomly select a sequence from $R_1$, $R_8$, $R_9$ and $R_{10}$ to update, and obtain a new nucleic acid sequence. Check whether the dissociation temperature of this nucleic acid sequence is greater than 52°C, and whether the free energy of the pairing region ( $\Delta G_S^\circ$ ) is less than -27.4 kcal/mol. If the constraint requirements of dissociation temperature and free energy of the pairing regions ( $\Delta G_S^\circ$ ) are not met, repeat the update. If the constraint requirements of dissociation temperature and free energy of the pairing region ( $\Delta G_S^\circ$ ) are met, update $S$ according to the principle of base complementation to obtain a new sequence set $S'$. If the new nucleic acid sequence obtained after fifteen updates does not meet the constraint requirements of dissociation temperature and pairing region free energy ( $\Delta G_S^\circ$ ), in order to prevent dead circulation, 5 becomes a new solution $S'$. Because this scheme uses a complete fixed core structure and part of tetramer sequences, during the process of generating a new solution, it is necessary to ensure that the fixed core structure and the retained tetramer sequences remain unchanged.

Optimization judgment: The same as the optimization judgment in Example 1. The difference is that the annealing initial temperature decays to 0.9 of the current value.

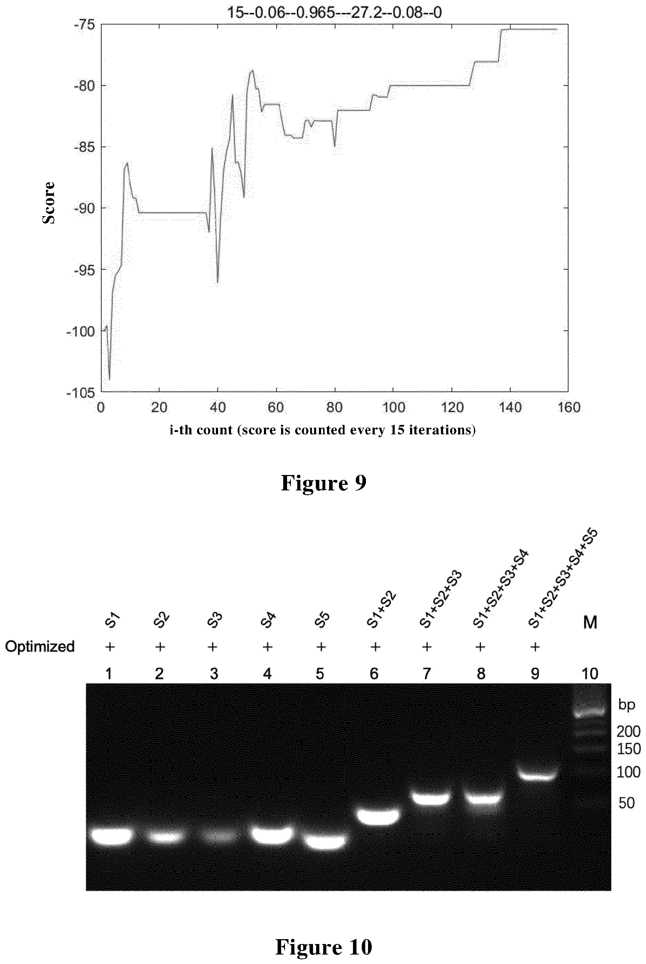

The optimized sequences of the first pentamer conversion scheme in Table 6_1 are obtained through this optimized algorithm, and Figure 9 shows the statistical line graph of the sum of the free energy values of the non-target pairing regions between the sequences during this optimization process (the free energy of the non-target pairing regions between $S_3$ and $S_3$, $S_3$ and $S_4$,$S_4$ and $S_4$ are not included in the statistics). Lane9 in Figure 10 shows the main band formed by the optimized sequence assembly, indicating the formation of pentamers and exhibiting high stability.

Table 6_1 Initialized sequences and optimized sequences for the first conversion scheme of pentamers

| Sequence numbering | Initialized sequences | SEQ ID NO: |
|---|---|---|
| $S_1$ | ATCACAGAGCGCGTAAAAACGCCACTCATGGA | 255 |
| $S_2$ | ATCCATGAGTGGCGTAAAAATCCAAATGAGCGTGTTACGG | 256 |
| $S_3$ | ACCGTAACACGCTCAAAACCGAAGTGCCAATT | 257 |
| $S_4$ | AAATTGGCACTTCGGAAAACTATGCGGCTGCT | 258 |
| $S_5$ | AAGCAGCCGCATAGTAAAGGATTAAATACGCGCTCTGTGA | 259 |
| Sequence numbering | Optimized sequences | |
| $S_1$ | ATTCAGGCGACTCCTAAAAGCACGACGATGGT | 260 |
| $S_2$ | AACCATCGTCGTGCTAAAAATCCAAATGAGCGTGTTACGG | 261 |
| $S_3$ | ACCGTAACACGCTCAAAACCGAAGTGCCAATT | 262 |
| $S_4$ | AAATTGGCACTTCGGAAAACTATGCGGCTGCT | 263 |
| $S_5$ | AAGCAGCCGCATAGTAAAGGATTAAAAGGAGTCGCCTGAA | 264 |

Table 6_2 Initialized sequence and optimized sequence free energy ($\Delta G_{NS}^{\circ}$) matrix for the first conversion scheme of pentamers

| Petramer initialized free energy matrix | | | | | | Pentamer optimized sequence free energy matrix | | | | | |
|---|---|---|---|---|---|---|---|---|---|---|---|
| Sequence | $S_1$ | $S_2$ | $S_3$ | $S_4$ | $S_5$ | Sequence | $S_1$ | $S_2$ | $S_3$ | $S_4$ | $S_5$ |
| $S_1$ | -10.36 | -8.09 | -9.92 | -8.16 | -6.75 | $S_1$ | -4.67 | -6.91 | -6.75 | -6.69 | -6.75 |
| $S_2$ | | -7.85 | -8.16 | -6.97 | -9.92 | $S_2$ | | -4.95 | -6.91 | -6.97 | -6.75 |
| $S_3$ | | | -5.36 | -6.75 | -6.75 | $S_3$ | | | -5.36 | -6.75 | -5.19 |
| $S_4$ | | | | -5.36 | -6.75 | $S_4$ | | | | -5.36 | -6.75 |
| $S_5$ | | | | | -10.36 | $S_5$ | | | | | -4.85 |

Table 6_3 Parameter indicators for pairing regions of pentamer optimized sequences 1

| Pairing sequences | CG% | $\Delta G_S^\circ$ | Tm °C (TF) | Tm °C (IDT) |
|---|---|---|---|---|
| $R_9$ | 57.1% | -27.63 | 53.2 | 48.8 |
| $R_1$ | 57.1% | -27.56 | 54.1 | 50.0 |
| $R_3$ | 53.6% | -27.42 | 53.0 | 48.0 |
| $R_5$ | 53.6% | -28.59 | 53.7 | 45.4 |
| $R_7$ | 57.1% | -28.57 | 53.6 | 50.2 |

The second conversion scheme: five nucleic acids that can be paired according to the shape shown in Figure 8(C) are designed. This scheme only retains the fixed core structure sequence of the tetramer. To adapt to the pentamer, the core structure is opened up at $R_1$ and $R_2$, the other parts need to be randomly generated and then optimized for the pentamer. Among them, nucleic acid single strands $R_{10}$, $R_2$, $R_4$, $R_6$ and $R_8$ are specifically complementary paired with nucleic acid single strands $R_1$, $R_3$, $R_5$, $R_7$ and $R_9$, respectively, without pairing with other nucleic acid single strands.

The free energy of specific complementary pairing ( $\Delta G_S^\circ$ ) is less than -27.4 kcal/mol, while the free energy of non-specific pairing ( $\Delta G_{NS}^\circ$ ) is greater than -7.2 kcal/mol. In this way, the form of pentamers is the most stable in the reaction system. The specific implementation steps for optimizing the second conversion scheme of pentamer are described in Figure 2; the annealing parameters are: annealing initial temperature $T_o$ = 50°C, annealing termination temperature $T_f$ = 0.12°C, and annealing temperature decay coefficient $\Delta T$ = 0.98 (each attenuation of annealing initial temperature is 0.98 of the current value); the optimized constraint parameters are: pairing sequence length $L$=14 bases, dissociation temperature threshold $T_m$ > 52°C, pairing sequence free energy threshold: $\Delta G_S^\circ < -27.4$ kcal/mol, and non-specific pairing free energy threshold: $\Delta G_{NS}^\circ > -7.2$ kcal/mol.

Table 7 Nucleic acid sequences $S_1$, $S_2$, $S_3$, $S_4$ and $S_5$ containing partial core structures

| $S_1$ | X1-Q1-AATCC-X2-R10-X3 |
|---|---|
| $S_2$ | X1-R2-X2-TGAGC-Q2-X3 |
| $S_3$ | X1-Q3-GCTCA-X2-CCGAA-Q4-X3 |
| $S_4$ | X1-Q5-TTCGG-X2-ACTAT-Q6-X3 |
| $S_5$ | X1-Q7-ATAGT-X2-GGATT-Q8-X3 |

The specific implementation steps for optimization are as follows:
Sequence initialization: according to the optimized constraint parameters, initialize the pairing sequence $R_9$ and the set $Q$ = {$Q_1$,$Q_3$, $Q_5$,$Q_7$} of sequences with a length of 9 except for the core structure. Based on the principle of base complementation, $R_8$ , $Q_2$ , $Q_4$ , $Q_6$ and $Q_8$ are obtained and concatenated according to Table 7, and finally the initialized sequence set $S$ = {$S_1$,$S_2$,$S_3$,$S_4$,$S_5$} is obtained.

Generation of new solution: the same as the generation of new solution in Example 1. The difference is that because this scheme uses a fixed core structure of tetramers, it is necessary to ensure that the fixed core structure remains partially unchanged during the generation of new solution. At the same time, constraint parameters need to be strictly followed, the dissociation temperature of the updated nucleic acid sequence pairing regions is greater than 52°C, and the free energy of the pairing regions ( $\Delta G_S^\varsigma$ ) is less than -27.4 kcal/mol.

Optimization judgment: the same as the optimization judgment in Example 1.

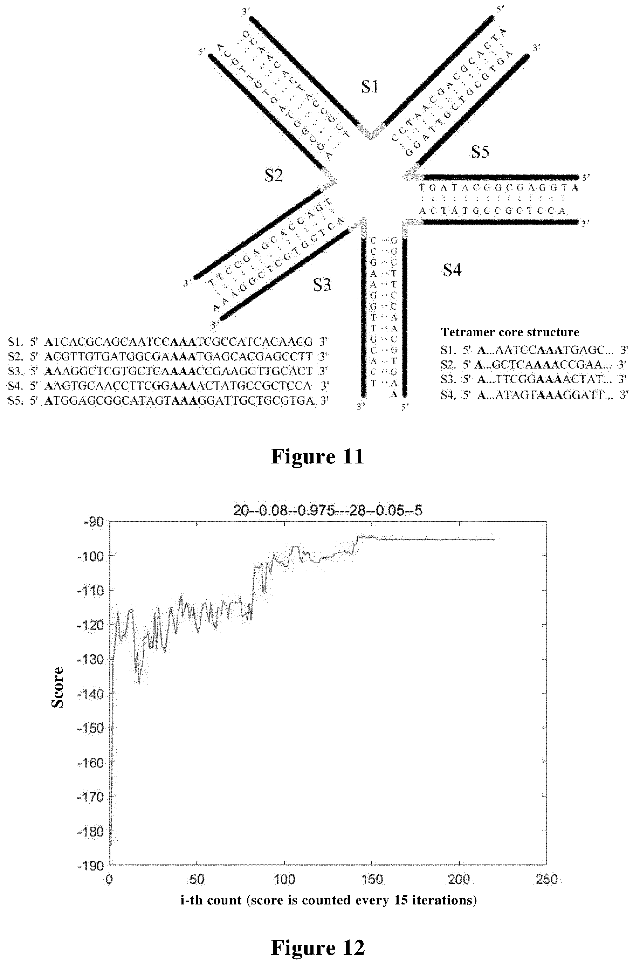

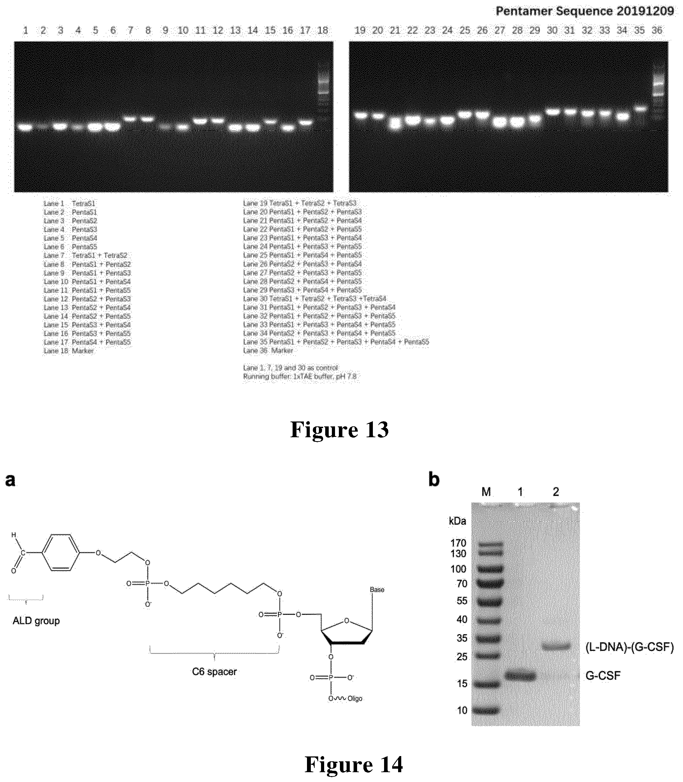

The optimized sequences of the second pentamer conversion scheme in Table 8_1 are obtained through this optimized algorithm, the specific pairing diagram thereof is shown in Figure 11, and Figure 12 shows the statistical line graph of the sum of free energy values of non-target pairing regions between sequences during this optimization process. In Figure 13, Lane35 is the main band formed by sequences $S_1$, $S_2$, $S_3$, $S_4$ and $S_5$. Although the pentamer shows somewhat trailing, the assembly effect is good.

Table 8_1 Initialized sequences and optimized sequences for the second conversion scheme of pentamers

| Sequence numbering | Initialized sequences | SEQ ID NO: |
|---|---|---|
| $S_1$ | ATGAGTGCGC**AATCC**AAATCGCCAGTCATGCA | 265 |
| $S_2$ | ATGCATGACTGGCGAAAA**TGAGC**GCTCGTTGA | 266 |
| $S_3$ | ATCAACGAGC**GCTCA**AAA**CCGAA**GTGCCAACT | 267 |
| $S_4$ | AAGTTGGCAC**TTCGG**AAA**ACTAT**CGCGCGACT | 268 |
| $S_5$ | AAGTCGCGCG**ATAGT**AAA**GGATT**GCGCACTCA | 269 |
| Sequence numbering | Optimized sequences | |
| $S_1$ | ATCACGCAGC**AATCC**AAATCGCCATCACAACG | 270 |
| $S_2$ | ACGTTGTGATGGCGA AAA**TGAGC**ACGAGCCTT | 271 |
| $S_3$ | AAAGGCTCGT**GCTCA**AAA**CCGAA**GGTTGCACT | 272 |
| $S_4$ | AAGTGCAACC**TTCGG**AAA**ACTAT**GCCGCTCCA | 273 |
| $S_5$ | ATGGAGCGGC**ATAGT**AAA**GGATT**GCTGCGTGA | 274 |

Table8_2 Initialized sequence and optimized sequence free energy ( $\Delta G_{NS}^{\circ}$ ) matrix for the second conversion scheme of pentamers

| Petramer initialized free energy matrix | | | | | | Pentamer optimized sequence free energy matrix | | | | | |
|---|---|---|---|---|---|---|---|---|---|---|---|
| Sequenc e | $S_1$ | $S_2$ | $S_3$ | $S_4$ | $S_5$ | Sequenc e | $S_1$ | $S_2$ | $S_3$ | $S_4$ | $S_5$ |
| $S_1$ | -13.79 | -9.89 | -9.89 | -9.89 | -9.89 | $S_1$ | -3.61 | -6.75 | -7.04 | -5.09 | -6.75 |
| $S_2$ | | -16.23 | -8.16 | -9.89 | -9.89 | $S_2$ | | -6.3 | -6.61 | -7.13 | -6.91 |
| $S_3$ | | | 16.2 3 | -9.89 | -9.89 | $S_3$ | | | -7.05 | -6.61 | -6.68 |
| $S_4$ | | | | -20.25 | 10.3 6 | $S_4$ | | | | -7.05 | -7.04 |
| $S_5$ | | | | | 20.2 5 | $S_5$ | | | | | -5.09 |

Table8_3 Parameter indicators for pairing regions of pentamer optimized sequences 2

| Pairing sequences | CG% | $\Delta G_S^\circ$ | Tm °C (TF) | Tm °C (IDT) |
|---|---|---|---|---|
| $R_1$ | 57.1 | -28.33 | 56.3 | 48.8 |
| $R_{10}$ | 57.1 | -28.55 | 57.4 | 48.8 |
| $R_3$ | 57.1 | -28.1 | 54.7 | 50 |
| $R_5$ | 57.1 | -28.14 | 53.8 | 48.3 |
| $R_7$ | 57.1 | -28.49 | 54.5 | 49.4 |

In addition, Examples 1, 2, and 3 are repeated to obtain the nucleic acid single strand sequences and sets thereof shown in Tables 9-1, 9-2, and 9-3 (see above) for forming the trimer, tetramer, and pentamer complexes based on the complementary nucleic acid backbone.

**Example 4: Coupling of G-CSF and L-DNA**

The coupling of G-CSF and L-DNA selectively couples L-DNA with aldehyde group modification at the 5' end to the N-terminal of G-CSF through a reductive amination reaction. Use dilution method or gel filtration chromatography and other methods to replace the buffer solution of G-CSF with acetate buffer solution (20 mM acetate, 150 mM NaCl, pH 5.0) and concentrate the sample to 30 mg/mL. Dissolve 100 OD (1 OD=33 μg) of L-DNA dry powder with aldehyde group modification at the 5' end in 60 μL acetate buffer. Take 30 mg of sodium cyanide borohydride, dissolve in acetate buffer, and adjust the concentration to 800 mM. Take 50 μL, 60 μL, and 20 μL of G-CSF, L-DNA, and sodium cyanide borohydride at the above concentrations, mix them evenly, and incubate them at room temperature in dark for 48 hours. The samples before and after the reaction are verified by polyacrylamide gel electrophoresis for the effect of coupling reaction. The (L-DNA)-(G-CSF) coupling compound deviated significantly compared to the uncoupled G-CSF on the electrophoresis gel figure, and the coupling efficiency can reach 70%~80% (Figure 14).

**Example 5: Purification of (L-DNA)-(G-CSF) coupling compound**

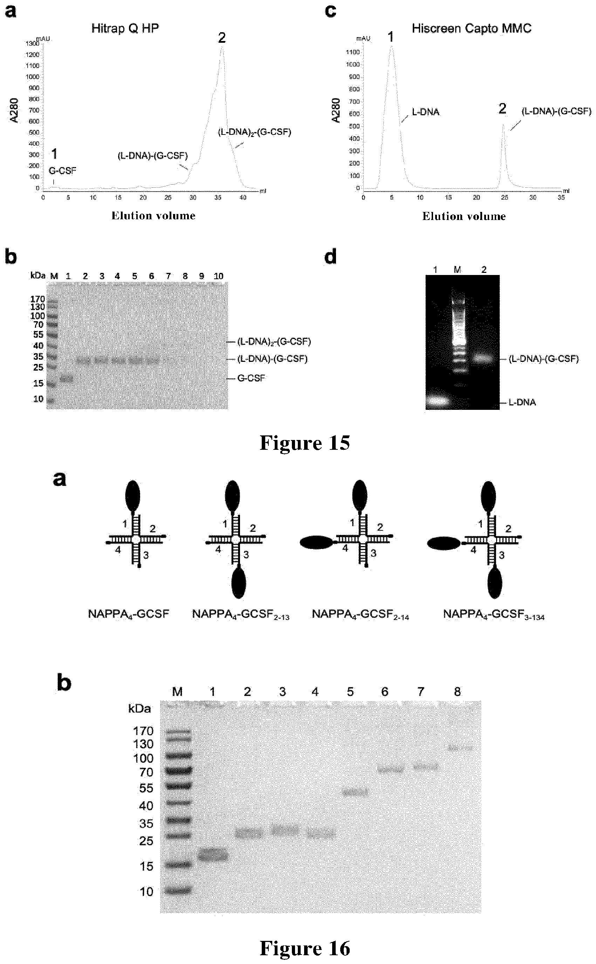

The purification of the (L-DNA)-(G-CSF) conjugate is divided into two steps. The first step is to remove the unreacted G-CSF and the $(L-DNA)_2$-(G-CSF) conjugate connected to two L-DNA strands using Hitrap Q HP (Figure 15a). The reaction mixture obtained in Example 5 is diluted 10 times with the loading buffer of Q column, and then loaded, eluted 10 times the column volume with the loading buffer to remove the unreacted G-CSF, and then eluted 50 times the column volume with a 0-100% linear gradient to separate the (L-DNA)-(G-CSF) coupling compound and $(L-DNA)_2$-(G-CSF) coupling compound, and identified the component type of each A280 absorption peak by polyacrylamide gel electrophoresis (Figure 15b). Collect the (L-DNA)-(G-CSF) conjugate (containing unreacted nucleic acids). The second step is to use Hiscreen Capto MMC to remove unreacted nucleic acids, and ultimately isolate the highly purified (L-DNA)-(G-CSF) conjugate (Figure 15c). Directly load the sample collected in step 1 onto the Hiscreen Capto MMC column, elute 10 times the column volume with the loading buffer to remove unreacted nucleic acids, and then elute the (L-DNA)-(G-CSF) conjugate with 100% elution buffer.

The purification conditions are as follows:

| Column | Loading buffer | Elution buffer | Gradient | Elution volume | Flow velocity |
|---|---|---|---|---|---|
| Hitrap Q HP | 25 mM acetate, pH 5.0 | 25 mM acetate, 1 M NaCl, pH 5.0 | 0-100% | 50倍柱体积 | 1 mL/min |
| Hiscreen Capto MMC | 25 mM acetate, pH 5.0 | 150 mM phosphate, 150 mM NaCl, pH 7.5 | 100% | 5倍柱体积 | 2 mL/min |

The purity of (L-DNA)-(G-CSF) conjugate sample obtained by two-step purification method is identified by 2% agarose gel electrophoresis (Fig. 15d). The gel diagram shows that there is only one nucleic acid band in the sample, and the (L-DNA)-(G-CSF) conjugate has a significant deviation compared to the uncoupled L-DNA on the gel diagram, indicating that the unreacted nucleic acid and (L-DNA)$_2$-(G-CSF) conjugate has been cleaned out.

**Example 6: Assembly of monovalent, divalent, and trivalent G-CSF complexes**

Measure the nucleic acid concentrations of S1-G-CSF, S3-G-CSF, S4-G-CSF, S2, S3, and S4 using Nanodrop. Take an appropriate amount of the above components according to the structural design of the monovalent, divalent, and trivalent protein complexes, and mix them in a 1:1:1:1 molar ratio. After mixing, each assembly unit automatically completes assembly according to the principle of base complementation. Before and after assembly, samples are identified by polyacrylamide gel electrophoresis for assembly effect and sample purity (Figure 16).

**Example 7: In vitro activity evaluation of G-CSF**

M-NFS-60 cells (mouse leukemia lymphocytes/G-CSF dependent cells) are inoculated into the resuscitation culture medium (RPMI1640+10% FBS+15 ng/mL G-CSF+1X penicillin-streptomycin), and the cells are resuscitated at 37°C and 5% $CO_2$ conditions. When the cell density reachs 80% - 90%, the cells are subcultured. After two or three times of subculture, the cells are inoculated into 96 well plates for cell plating. The cell plating experiment uses coming 3599 # 96 well plates, with a cell plating density of 6000 cells per well. Gradient dilution is performed on different samples (GCSF, NAPPA$_4$-GCSF, NAPPA$_4$-GCSF$_2$, NAPPA$_4$-GCSF$_3$) at working concentrations of (0.001, 0.01, 0.1, 1, 10, 100 ng/mL), with a final volume of 100 $\mu$L. Use PBS as a control. After incubating in a constant temperature incubator for 48 hours, add 10 $\mu$L of CCK8 solution to each well, and incubate the culture plate in the incubator for 1-4 hours, measure the absorbance at 450 nm using an enzyme-linked immunosorbent assay, and calculate the cell proliferation rate of different samples.

$$\text{Cell proliferation rate (\%)} = [A\ (dosing) - A\ (0\ dosing)]/[A\ (0\ dosing) - A\ (blank)] \times 100$$

A (dosing): absorbance of wells with cells, CCK solution, and drug solution
A (blank): absorbance of wells with culture medium and CCK8 solution without cells
A (0 dosing): absorbance of wells with cells, CCK8 solution, without drug solution

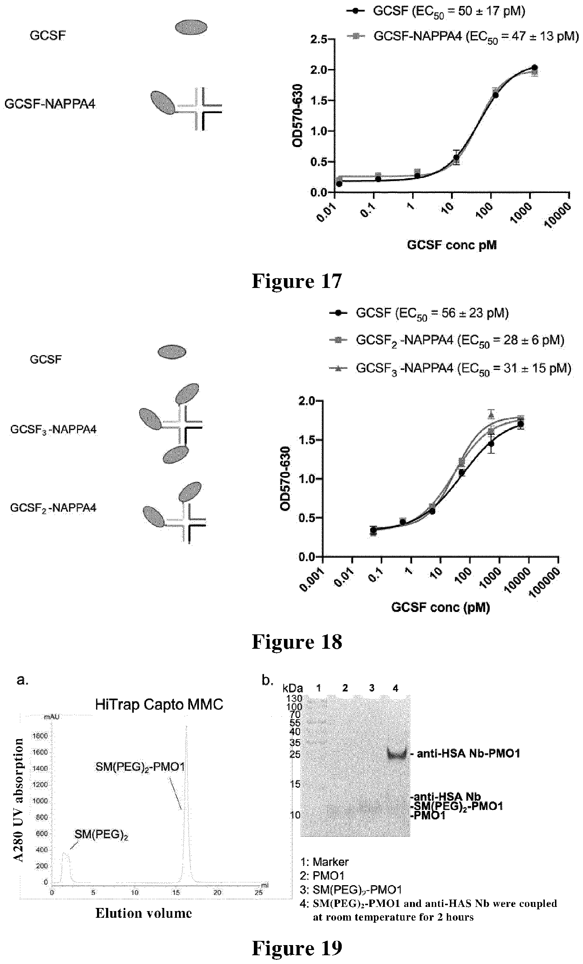

The activity of a G-CSF linked L-DNA tetramer framework is evaluated using the above activity testing method, and it is found that the L-DNA tetramer framework has no effect on the activity of G-CSF (Figure 17). Using the same activity testing method, it is found that bivalent and trivalent G-CSFs assembled with L-DNA tetramers do not have a negative impact on the activity of G-CSF (Figure 18).

**Example 8: Coupling and purification of SM(PEG)$_2$-PMO coupling compound**

In this embodiment, phosphorodiamidate morpholino nucleic acid is used for the experiment. Specifically, the following four PMO single strand sequences are selected (from 5' to 3')

Strand 1 (PMOl): SEQ ID NO: 275 5'- AGCAGCCTCGTTGAATCGCCAAGACACC -3'
Strand 2 (PMO2): SEQ ID NO: 276 5'- AGGTGTCTTGGCGAAAGTTGCTCCGACG -3'
Strand 3 (PMO3): SEQ ID NO: 277 5'- ACGTCGGAGCAACTAAGCGGTTCTGTGG -3'
Strand 4 (PMO3): SEQ ID NO: 278 5'- ACCACAGAACCGCTATCAACGAGGCTGC -3'

The 5' end is modified with an NH$_2$ group, which is used for coupling the NHS active group of SM(PEG)$_2$.
Dissolve PMO single strand containing 5'-terminal NH$_2$ modification with phosphate buffer (50 mM NaH$_2$PO$_4$, 150 mM NaCl, pH 7.4) to prepare a mother solution with a final concentration of 1 mM. Dissolve SM(PEG)$_2$ (linker molecule) powder with dimethyl sulfoxide (DMSO) and freshly prepare 250 mM of SM(PEG)$_2$ mother solution. Add 10 to 50 times molar amounts of SM(PEG)$_2$ mother solution to the PMO single strand mother solution, quickly mix them, and react at room temperature for 30 minutes to 2 hours. After the reaction is completed, add 10% volume of 1M Tris HCl (pH 7.0) to the reaction solution, mix them and incubate at room temperature for 20 minutes to quench the excessive SM(PEG)$_2$ reaction. After incubation, the SM(PEG)$_2$-PMO is purified using Hitrap Capto MMC. Unreacted SM(PEG)$_2$ flows through the column without binding, while SM(PEG)$_2$-PMO bound to the upper column is eluted with buffer (25 mM BICINE, 200 mM NH$_4$Cl, 1M Arginine monohydrochloride, pH 8.5). The elution results are shown in Figure 19a.

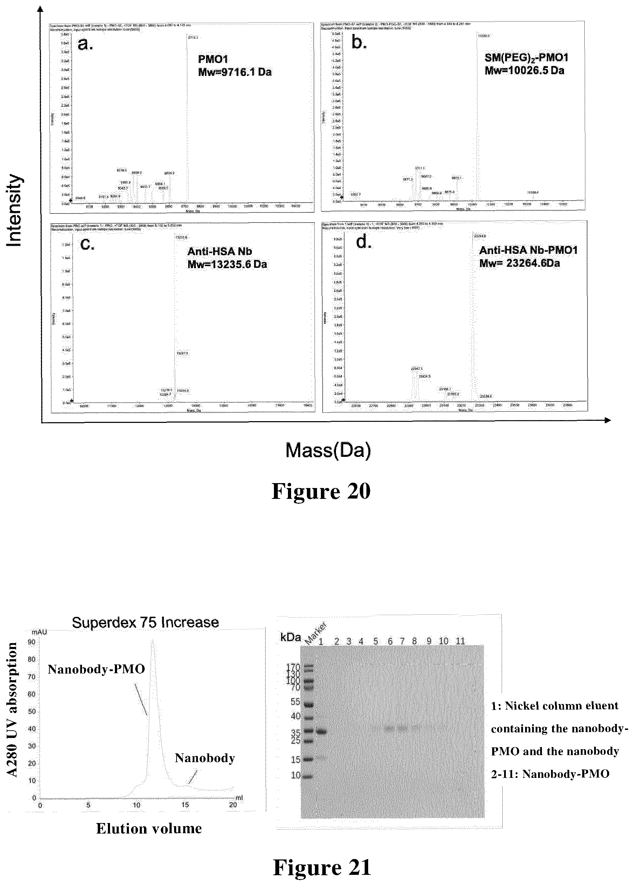

Analyze PMO samples before and after coupling using a positive ion mode of liquid chromatography-mass spectrometry. As shown in Figure 20a and Figure 20b, the results show that the final SM(PEG)$_2$-PMO molecular weight is consistent with the theoretical value, and the coupling reaction efficiency is high.

**Example 9: Preparation of nano antibody mutants**

Cysteine mutations are introduced into the carboxyl end of nano antibodies for nucleic acid coupling. Optimize the gene sequence of the anti-HSA nano antibody into the yeast preferred codons, and then subclone it into the pPICZ alpha A plasmid. The amino acid sequence of the anti-HSA nano antibody is shown in SEQ ID NO: 279. To facilitate purification, the N-terminal of the nano antibody is labeled with His.

SEQ ID NO: 279, the amino acid sequence of the anti-HSA nano antibody:

HHHHHHAVQLVESGGGLVQPGNSLRLSCAASGFTFRSFGMSWVR
QAPGKEPEWVSSISGSGSDTLYADSVKGRFTISRDNAKTTLYLQMNSLK
PEDTAVYYCTIGGSLSRSSQGTQVTVSSGSC

Linearize the plasmid and transfer it to Pichia pastoris strain X33, and screen the high copy strain of the target gene using Zeocin concentration gradient YPD agar plate. Cultivate monoclonal strains using GMGY at 30°C and 250 rpm conditions to obtain sufficient strains. Then, induce the expression and secretion of target nano antibodies using GMMY at 20°C and 250 rpm conditions, and supplement with 1% methanol every 24 hours.

The expression yield of nano antibodies in laboratory grade glass flasks of the high copy-screened strain can reach 40-80 mg/L.

After SDS-PAGE identification and analysis, the culture supernatant after 72 hours of induction contains a large number of target nano antibody monomers and nano antibody dimers. Purify the nano antibodies in the culture supernatant using His labeled affinity column.

**Example 10: Coupling and purification of nano antibody-PMO conjugates**

Dialyze the nano antibody sample eluted by His label affinity chromatography (Example 9) with a dialysis buffer containing a reducing agent (20 mM Tris, 15 mM NaCl, pH 7.4). During the dialysis process, the C-terminal thiol group is reduced while removing the small impurities such as free -SH groups. Mix the reduced nano antibody with SM(PEG)$_2$-PMO single strand (prepared in Example 8) in a molar ratio of 1:1 to 2, and react at room temperature for 2 hours after mixing evenly.

As shown in Figure 19b, the coupling efficiency can reach over 90% through SDS-PAGE identification.

Using His labeled affinity column to remove unreacted SM(PEG)$_2$-PMO single strands, nano antibodies and nano antibody-PMO mixtures are collected.

Using Superdex™ 75 Increase 10/300 GL to separate nano antibodies and nano antibody-PMO, and the nano antibodies and nano antibody-PMO are effectively separated as shown in Figure 21.

The nano antibodies before and after nucleic acid coupling are analyzed using a positive ion mode of liquid chromatography-mass spectrometry. As shown in Figures 20c and d, the final nano antibody-PMO molecular weight obtained is consistent with the theoretical value.

**Example 11: Self-assembly of NAPPA-PMO drugs**

Taking pmo NAPPA4-HSA (1) as an example, the self-assembly process of NAPPA-PMO drugs is introduced below.

Measure the concentrations of anti-HSA Nb-PMO1, PMO2, PMO3, and PMO4 respectively. Take an appropriate amount of the above components and preheat at 37 °C for 5 minutes, then mix them in a 1:1 molar ratio at 37 °C condition and incubate for 1 minute. Complete the assembly of pmo-NAPPA4-HSA (1).

Similarly, the pmo-NAPPA4-HSA (1,2,3) is assembled, and the required modules are anti-HSA Nb-PMO1, anti-HSA Nb-PMO2, anti-HSA Nb-PMO3, and PMO4.

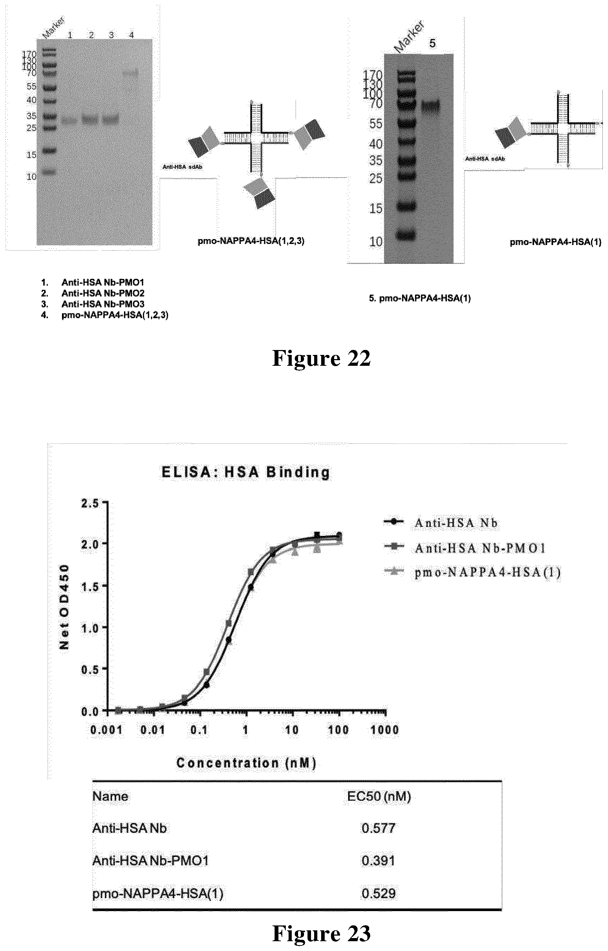

Under low temperature conditions, the assembly of the samples is identified using SDS-PAGE. As shown in Figure 22, the results show that the assembled samples have uniform bands.

**Example 12: Verification of binding activity of nano antibody-PMO monomers and assembled NAPPA-PMO drugs**

Taking pmo-NAPPA4-HSA (1) as an example, the binding ability of anti-HSA nano antibody, anti-HSA Nb-PMO1 monomer and assembled pmo-NAPPA4-HSA (1) to HSA protein (ACRO Biosystems, HSA-H5220) is tested by ELISA.

Each well of the 96-well ELISA plate is coated with 100 ng of HSA protein overnight at 4 °C. Wash the plate with washing solution (PBS containing 0.05% Tween-20) and block it with blocking solution (PBS containing 3% BSA and 0.05% Tween-20), then add gradient diluted Anti-HSA nano antibodies, nano antibodies coupled with PMO, Anti-HSA Nb-PMO1, and assembled pmo-NAPPA4-HSA (1), and incubate at room temperature for 1 hour. After washing three times, add 1:5000 diluted horseradish peroxidase coupled rabbit-anti-camel VHH antibody (Genscript, A02016) and incubate at room temperature for 1 hour. After washing 3 times, add a tetramethylbenzidine substrate solution (Biyuntian, P0209) for development. Use a termination solution (Biyuntian, P0215) to quench the development. Use an enzyme-linked immunosorbent assay (MolecularDevices, SpectraMax i3x) to read the absorbance at 450 nm for each well and calculate the corresponding EC50.

The calculation results in Figure 23 show that the EC50 of the binding of Anti-HSA nano antibodies, anti-HSA Nb-PMO1, and assembled pmo-NAPPA4-HSA (1) to HSA protein are 0.577 nM, 0.391 nM, and 0.529 nM, respectively. This indicates that the PMO coupling method of nano antibodies does not affect the binding activity of nano antibodies to corresponding antigens, and the PMO assembly method also does not affect the binding activity of nano antibodies to corresponding antigens.

**Example 13: Resistance experiment of NAPPA-PMO drugs to nuclease degradation**

PMO, as a nucleic acid derivative, can withstand the degradation of various nuclease. In order to verify whether the assembled NAPPA-PMO drugs can also tolerate nuclease or depolymerization, the following experiment is designed. Three common nuclease DNase I (Thermo Scientific, EN0523), T7 Endonuclease I (NEB, M0302S) and S1 Nuclease (Thermo Scientific, EN0321) are selected to incubate the PMO assembly sample pmo-NAPPA4-HSA (1) and the D-DNA assembly sample DDNA-NAPPA4 (control) for one hour at 37 °C. The incubated pmo NAPPA4-HSA (1) and DDNA-NAPPA4 are analyzed using SDS-PAGE and 2% agarose electrophoresis, respectively.

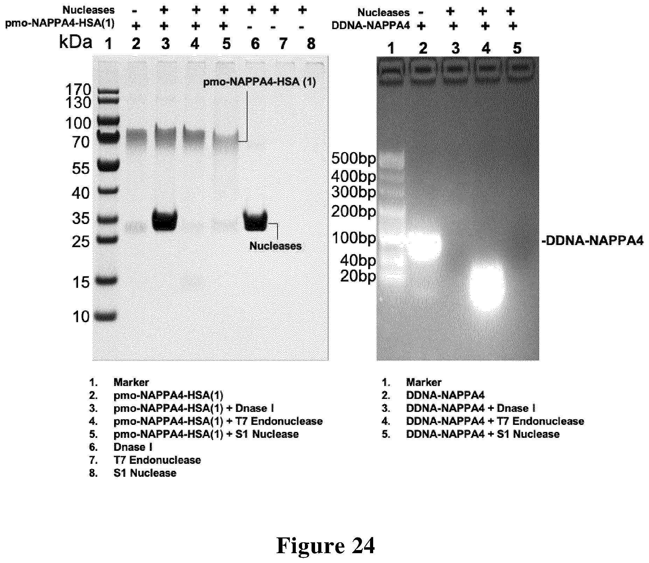

As shown in Figure 24, the pmo-NAPPA4-HSA(1) is not degraded by the three nuclease (left figure), while DDNA-NAPPA4 is completely degraded by DNase I and S1 Nuclease, and cut into shorter fragments by T7 Endonuclease I. Therefore, the experiment shows that NAPPA-PMO drugs can tolerate the degradation of common nuclease.

All documents mentioned in the present invention are incorporated by reference herein as if each document was incorporated separately by reference. Furthermore, it should be understood that after reading the foregoing teachings of the present invention, various changes or modifications can be made to the present invention by those skilled in the art and that these equivalents also fall in the scope of the claims appended to this application.

**Claims**

1. A multimeric complex based on a complementary nucleic acid backbone, wherein the complex is a multimer formed by complexing n monomers having the complementary nucleic acid backbone, wherein each monomer is a polypeptide having a nucleic acid single strand, and n is a positive integer of 3-6; in the multimer, the nucleic acid single strand of each monomer and the nucleic acid single strands of the other two monomers form complementary double strands by means of base complementation, so as to form complementary nucleic acid backbone structures.

2. The multimeric complex of claim 1, wherein the monomer has a structure of formula I:

Z1-W        (I)

wherein,

Z1 is a polypeptide moiety;
W is a nucleic acid single strand sequence; and
"-" is a linker or bond.

3. The multimeric complex of claim 2, wherein the nucleic acid sequence W has the structure shown in formula 1:

X1-R1-X2-R2-X3        (1)

wherein,

> R1 is a complementary base pairing region 1;
> R2 is a complementary base pairing region 2;
> Each of X1, X2, and X3 is independently not present or redundant nucleic acids; and
> "-" is a bond.

4. The multimeric complex of claim 2, wherein the sequence of X2 is selected from the group consisting of: A, AA, AGA and AAA.

5. The multimeric complex of any one of claims 1-4, wherein the monomer sequence is any sequence or a sequence set thereof selected from the nucleic acid single strand sequences as shown in SEQ ID Nos: 1-60 that form a trimer complex based on the complementary nucleic acid backbone.

6. The multimeric complex of any one of claims 1-4, wherein the monomer sequence is any sequence or a sequence set thereof selected from the nucleic acid single strand sequences as shown in SEQ ID Nos: 61-140 that form a tetramer complex based on the complementary nucleic acid backbone.

7. The multimeric complex of any one of claims 1-4, wherein the monomer sequence is any sequence or a sequence set thereof selected from the nucleic acid single strand sequences as shown in SEQ ID Nos: 141-240 that forms a pentamer complex based on the complementary nucleic acid backbone.

8. The multimeric complex of any one of claims 1-4, wherein the monomer sequence is any sequence or a sequence set thereof selected from the nucleic acid single strand sequences as shown in SEQ ID Nos: 275-278 that forms a tetramer complex based on the complementary nucleic acid backbone.

9. A pharmaceutical composition comprising:

> (a) the multimeric complex based on the complementary nucleic acid backbone of claim 1; and
> (b) a pharmaceutically acceptable carrier.

10. A nucleic acid sequence library, which comprises a nucleic acid sequence for forming the multimeric complex based on the complementary nucleic acid backbone of claim 1.

11. The nucleic acid sequence library of claim 10, wherein the nucleic acid sequence W has the structure shown in formula 1:

$$X1-R1-X2-R2-X3 \qquad (1)$$

wherein,

> R1 is the complementary base pairing region 1;
> R2 is the complementary base pairing region 2;
> Each of X1, X2, and X3 is independently not present or redundant nucleic acids; and
> "-" is a bond.

12. Use of the nucleic acid sequence library of claim 10 in the manufacture of the multimeric complex of claim 1 or a pharmaceutical composition comprising the multimeric complex of claim 1.

13. A method of determining a nucleic acid single strand sequence for forming a multimeric complex based on a complementary nucleic acid backbone, comprising steps of:

> (a) setting annealing algorithm parameters:

> > setting the initial annealing temperature, annealing termination temperature, and annealing temperature attenuation coefficient $\Delta T$;
> > setting optimized constraint parameters:

①the number n of the nucleic acid single strand, preferably a positive integer of 3-6;

②the length $L$ of the pairing sequence, preferably the $L$ is of 12-16 bases;

③the dissociation temperature threshold $T_m$ of the pairing region;

④the free energy threshold $\Delta G_S^{\circ}$ of the specific pairing region sequence;

⑤the free energy threshold $\Delta G_{NS}^{\circ}$ of the non-specific pairing;

⑥the connecting element X2, preferably A, AA, and AAA;

⑦the dissociation temperature threshold $T_{m\text{-}H}$ of the secondary structure (hairpin);

⑧the CG proportion $P_{CG}$ in the pairing sequence, preferably the range of $P_{CG}$ is [0.4,0.6];

⑨optionally, for n=4, using a symmetric sequence to initialize a sequence set $S = \{S_1, S_2, \dots , S_n\}$ according to the above parameters;

(b) calculating the objective function value $E_0$ of the set $S$ of the previous step, that is, calculating the sum of the non-specific pairing free energies ( $\Delta G_{NS}^{\circ}$ ) between sequences and of the sequence itself, while obtaining the non-specific pairing free energy matrix $C_{n \times n}$, searching the $S_i$ and $S_j (1 \leq i \leq n, 1 \leq j \leq n)$ corresponding to the minimum value in the upper triangular matrix thereof, randomly selecting $S_i$ or $S_j$ for an updated operation according to the non-specific pairing free energy of the $S_i$ and $S_j$ $\Delta G_{NS}^{\circ}(S_i, S_j)$ , and then obtaining a new nucleic acid sequence, thereby obtaining a updated sequence set S';

(c) determining whether the sequences in the set $S'$ of the previous step meet the optimized constraint parameter conditions set in step (a), verifying the following parameters, including the dissociation temperature $T_m$ of the specific pairing region, the free energy $\Delta G_S^{\circ}$ of the specific pairing region sequence, the dissociation temperature $T_{m\text{-}H}$ of the secondary structure and the CG proportion $P_{CG}$. If the above parameters meet the constraint conditions, the step (d) is proceeded; otherwise, the step (c) is repeated. If the step (b) is performed 15 times continuously at a certain annealing temperature without obtaining the $S'$ that meets the conditions, then the set S becomes the set $S'$ and the next step is proceeded to prevent a dead cycle;

(d) calculating the objective function value $E_1$ of the set $S'$ of the previous step, and comparing $E_0$ with $E_1$. If $E_1 \geq E_0$, it indicates that the non-specific pairing free energy has been optimized, and the sequence set $S'$ becomes the sequence set $S$. If $E_1 < E_0$, it indicates that the non-specific pairing free energy has not been optimized, and in this case, it is necessary to determine whether to accept the set $S'$ as $S$ according to the Metropolis criterion; and

(e) the annealing temperature is attenuated according to the attenuation coefficient $\Delta T$ set in the step (a), and the steps (b), (c), and (d) are repeated for the $S$ of the previous step, which is the Monte Carlo-based annealing algorithm, until the annealing temperature reaches the annealing termination temperature. The $S = \{S_1, S_2, \dots , S_n\}$ of the previous step becomes the nucleic acid single strand sequence for forming the multimeric complex based on the complementary nucleic acid backbone.

**14.** A nucleic acid single strand sequence set for forming a multimeric complex based on a complementary nucleic acid backbone, which is determined using the method of claim 13.

**15.** The nucleic acid single strand sequence set of claim 14, wherein the set is selected from the group consisting of:

(S1) a nucleic acid single strand sequence for forming a trimer complex based on the complementary nucleic acid backbone:

| Sequence set 3-1 numbering | Optimized sequence | SEQ ID NO: |
|---|---|---|
| $S_1$ | $\underline{A}$CACCTGGTTGTTGGAT$\underline{AAA}$TCGTTGAAG GCTAGGA | 1 |
| $S_2$ | $\underline{A}$TCCTAGCCTTCAACGA$\underline{AAA}$AACTAGAGT CCGCCGA | 2 |

(continued)

| Sequence set 3-1 numbering | Optimized sequence | SEQ ID NO: |
|---|---|---|
| $S_3$ | ATCGGCGGACTCTAGTTAAAATCCAACAACCAGGTG | 3 |
| Sequence set 3-2 numbering | Optimized sequence | |
| $S_1$ | ATGCGTTGAGTTCCAGTAAAGGCAACATCACCACAT | 4 |
| $S_2$ | AATGTGGTGATGTTGCCAAATCTGAATCCTCGTGCT | 5 |
| $S_3$ | AAGCACGAGGATTCAGAAAAACTGGAACTCAACGCA | 6 |
| Sequence set 3-3 numbering | Optimized sequence | |
| $S_1$ | ATTCCAATCGTCCTGTGAAAAGTTCCGCTCTGAGTT | 7 |
| $S_2$ | AAACTCAGAGCGGAACTAAACTGGCAGATGGATGAA | 8 |
| $S_3$ | ATTCATCCATCTGCCAGAAACACAGGACGATTGGAA | 9 |
| Sequence set 3-4 numbering | Optimized sequence | |
| $S_1$ | ACGAGGCAAGTTCTGTGAAAATGACTACCAGGTCCG | 10 |
| $S_2$ | ACGGACCTGGTAGTCATAAAATCCACTGACGCTGAA | 11 |
| $S_3$ | ATTCAGCGTCAGTGGATAAACACAGAACTTGCCTCG | 12 |
| Sequence set 3-5 numbering | Optimized sequence | |
| $S_1$ | ATAGTTCGTTGCTCGGAAAAGGCATTGAGAGGACCT | 13 |
| $S_2$ | AAGGTCCTCTCAATGCCAAAATGGTGATGTCGCTTG | 14 |
| $S_3$ | ACAAGCGACATCACCATAAATCCGAGCAACGAACTA | 15 |
| Sequence set 3-6 numbering | Optimized sequence | |
| $S_1$ | AGTCGTGTGCTTCCAAGAAATAGCCAGGTGAGGACT | 16 |
| $S_2$ | AAGTCCTCACCTGGCTAAAAAACAGCGGAGTGTCAT | 17 |

(continued)

| Sequence set 3-6 numbering | Optimized sequence | |
|---|---|---|
| $S_3$ | AATGACACTCCGCTGTTAAACTTGGAAGC ACACGAC | 18 |
| Sequence set 3-7 numbering | Optimized sequence | |
| $S_1$ | AACGCATCGCTTGATAGAAAAGAGGAGC ACGGTTAT | 19 |
| $S_2$ | AATAACCGTGCTCCTCTAAAGTAGGCAAT CCACCAT | 20 |
| $S_3$ | AATGGTGGATTGCCTACAAACTATCAAGC GATGCGT | 21 |
| Sequence set 3-8 numbering | Optimized sequence | |
| $S_1$ | AGTCGTTCCACCGAACAAAATGGCTCTGG TCATTGA | 22 |
| $S_2$ | ATCAATGACCAGAGCCAAAAAATCGCAC ATCTCAGG | 23 |
| $S_3$ | ACCTGAGATGTGCGATTAAATGTTCGGTG | 24 |
| | GAACGAC | |
| Sequence set 3-9 numbering | Optimized sequence | |
| $S_1$ | AGCGGAGTGACCATAGTAAAAGGCAGGA CATTGTTC | 25 |
| $S_2$ | AGAACAATGTCCTGCCTAAAGTGCTCGTC GTGAAGA | 26 |
| $S_3$ | ATCTTCACGACGAGCACAAACTATGGTC ACTCCGC | 27 |
| Sequence set 3-10 numbering | Optimized sequence | |
| $S_1$ | AATTGGACCGCTCTACTAAAATGGCACCA CAGTCAA | 28 |
| $S_2$ | ATTGACTGTGGTGCCATAAACAGGCTATC AGCATCC | 29 |
| $S_3$ | AGGATGCTGATAGCCTGAAAAGTAGAGC GGTCCAAT | 30 |
| Sequence set 3-11 numbering | Optimized sequence | |
| $S_1$ | ACCATTGAGCCAGTGATAAAAACCGTTGT GAGTTGC | 31 |
| $S_2$ | AGCAACTCACAACGGTTAAATCGCACACC TGTCGTA | 32 |

(continued)

| Sequence set 3-11 numbering | Optimized sequence | |
|---|---|---|
| $S_3$ | ATACGACAGGTGTGCGAAAAATCACTGGCTCAATGG | 33 |
| Sequence set 3-12 numbering | Optimized sequence | |
| $S_1$ | AAGTGAAGAAGCAGCCTAAAGTTGTCATCGCACACC | 34 |
| $S_2$ | AGGTGTGCGATGACAACAAAATGTCGTAACCGTGGA | 35 |
| $S_3$ | ATCCACGGTTACGACATAAAAGGCTGCTTCTTCACT | 36 |
| Sequence set 3-13 numbering | Optimized sequence | |
| $S_1$ | AATAGCGTCTTGAGCCTAAATGGAGGACATACCGAC | 37 |
| $S_2$ | AGTCGGTATGTCCTCCAAAAGGTCACAGTTGCTGCT | 38 |
| $S_3$ | AAGCAGCAACTGTGACCAAAAGGCTCAAGACGCTAT | 39 |
| Sequence set 3-14 numbering | Optimized sequence | |
| $S_1$ | ATGCCGTGTTCAGATTCAAATGTGCGTCTGGATTGA | 40 |
| $S_2$ | ATCAATCCAGACGCACAAAAAGACAGGTGGTCCGAT | 41 |
| $S_3$ | AATCGGACCACCTGTCTAAAGAATCTGAACACGGCA | 42 |
| Sequence set 3-15 numbering | Optimized sequence | |
| $S_1$ | ATTCAGGACAGCGTCATAAAACCGACTGGAGCAACT | 43 |
| $S_2$ | AAGTTGCTCCAGTCGGTAAAGATGCCTTCGTGTGAG | 44 |
| $S_3$ | ACTCACACGAAGGCATCAAAATGACGCTGTCCTGAA | 45 |
| Sequence set 3-16 numbering | Optimized sequence | |
| $S_1$ | AGCAGCCAAGGTTATCTAAACAATGACACGGAGGAT | 46 |
| $S_2$ | AATCCTCCGTGTCATTGAAAGTGATTCGCACCAGAC | 47 |

(continued)

| Sequence set 3-16 numbering | Optimized sequence | |
|---|---|---|
| $S_3$ | AGTCTGGTGCGAATCACAAAAGATAACCTTGGCTGC | 48 |
| Sequence set 3-17 numbering | Optimized sequence | |
| $S_1$ | ACCACCGTGTATGACCTAAAAGTGACAGCACATCGC | 49 |
| $S_2$ | AGCGATGTGCTGTCACTAAAACAGGCTCTACGAGGA | 50 |
| $S_3$ | ATCCTCGTAGAGCCTGTAAAAGGTCATACACGGTGG | 51 |
| Sequence set 3-18 numbering | Optimized sequence | |
| $S_1$ | AACTACGGAGCGAAGATAAATCCTGACCAACTTGCT | 52 |
| $S_2$ | AAGCAAGTTGGTCAGGAAAAGACTGGCTGAACACGA | 53 |
| $S_3$ | ATCGTGTTCAGCCAGTCAAAATCTTCGCTCCGTAGT | 54 |
| Sequence set 3-19 numbering | Optimized sequence | |
| $S_1$ | AGTTCCTGATCCAGCCTAAACATCCTTGTCTTGCCA | 55 |
| $S_2$ | ATGGCAAGACAAGGATGAAACACGACCGCTTAGAAG | 56 |
| $S_3$ | ACTTCTAAGCGGTCGTGAAAAGGCTGGATCAGGAAC | 57 |
| Sequence set 3-20 numbering | Optimized sequence | |
| $S_1$ | ATATCGCACTCCAGCATAAACCGTGTGAACATCAGG | 58 |
| $S_2$ | ACCTGATGTTCACACGGAAAAGCCTACGAGACTTGG | 59 |
| $S_3$ | ACCAAGTCTCGTAGGCTAAAATGCTGGAGTGCGATA | 60 |

(S2) a nucleic acid single strand sequence for forming a tetramer complex based on the complementary nucleic acid backbone:

| Sequence set 4-1 numbering | Optimized sequence | SEQ ID NO: |
|---|---|---|
| $S_1$ | AAGCGTCGTGAATCCAAATGAGCCTGCCAATG | 61 |

(continued)

| Sequence set 4-1 numbering | Optimized sequence | SEQ ID NO: |
|---|---|---|
| $S_2$ | <u>A</u>CATTGGCAGGCTCA<u>AAA</u>CCGAAGTCAACGCT | 62 |
| $S_3$ | <u>A</u>AGCGTTGACTTCGG<u>AAA</u>ACTATGGACGGCGA | 63 |
| S4 | <u>A</u>TCGCCGTCCATAGT<u>AAA</u>GGATTCACGACGCT | 64 |
| Sequence set 4-2 numbering | Optimized sequence | |
| $S_1$ | <u>A</u>ATGGCGAGCAATCC<u>AAA</u>TGAGCCTGGACCAA | 65 |
| $S_2$ | <u>A</u>TTGGTCCAGGCTCA<u>AAA</u>CCGAACGCTGTGAT | 66 |
| $S_3$ | <u>A</u>ATCACAGCGTTCGG<u>AAA</u>ACTATCGTGCGGCA | 67 |
| $S_4$ | <u>A</u>TGCCGCACGATAGT<u>AAA</u>GGATTGCTCGCCAT | 68 |
| Sequence set 4-3 numbering | Optimized sequence | |
| $S_1$ | <u>A</u>TGACCACGCAATCC<u>AAA</u>TGAGCCAACCTCCA | 69 |
| $S_2$ | <u>A</u>TGGAGGTTGGCTCA<u>AAA</u>CCGAACAGCAGCTT | 70 |
| $S_3$ | <u>A</u>AAGCTGCTGTTCGG<u>AAA</u>ACTATCTGCCGCCT | 71 |
| $S_4$ | <u>A</u>AGGCGGCAGATAGT<u>AAA</u>GGATTGCGTGGTCA | 72 |
| Sequence set 4-4 numbering | Optimized sequence | |
| $S_1$ | <u>A</u>TGTCGCACCAATCC<u>AAA</u>TGAGCAAGCCTCGT | 73 |
| $S_2$ | <u>A</u>ACGAGGCTTGCTCA<u>AAA</u>CCGAACGCTGTCAT | 74 |
| $S_3$ | <u>A</u>ATGACAGCGTTCGG<u>AAA</u>ACTATGTGGCGGCA | 75 |
| $S_4$ | <u>A</u>TGCCGCCACATAGT<u>AAA</u>GGATTGGTGCGACA | 76 |
| Sequence set 4-5 numbering | Optimized sequence | |
| $S_1$ | <u>A</u>TGCTGGCACAATCC<u>AAA</u>TGAGCGACGAGGTT | 77 |

(continued)

| Sequence set 4-5 numbering | Optimized sequence | |
|---|---|---|
| $S_2$ | AAACCTCGTCGCTCAAAACCGAAGTGCCAGTT | 78 |
| $S_3$ | AAACTGGCACTTCGGAAAACTATGAGGCGGCT | 79 |
| $S_4$ | AAGCCGCCTCATAGTAAAGGATTGTGCCAGCA | 80 |
| Sequence set 4-6 numbering | Optimized sequence | |
| $S_1$ | ATGTCGCACCAATCCAAATGAGCAGGTTGGCA | 81 |
| $S_2$ | ATGCCAACCTGCTCAAAACCGAACGCTGTCAA | 82 |
| $S_3$ | ATTGACAGCGTTCGGAAAACTATCAGCCGCCT | 83 |
| $S_4$ | AAGGCGGCTGATAGTAAAGGATTGGTGCGACA | 84 |
| Sequence set 4-7 numbering | Optimized sequence | |
| $S_1$ | ATGTGGTCGCAATCCAAATGAGCACCTGCCAA | 85 |
| $S_2$ | ATTGGCAGGTGCTCAAAACCGAACGTGACGAT | 86 |
| $S_3$ | AATCGTCACGTTCGGAAAACTATCAACGCCGC | 87 |
| $S_4$ | AGCGGCGTTGATAGTAAAGGATTGCGACCACA | 88 |
| Sequence set 4-8 numbering | Optimized sequence | |
| $S_1$ | AAGCGTCGTCAATCCAAATGAGCACGGCAATG | 89 |
| $S_2$ | ACATTGCCGTGCTCAAAACCGAAGTGAACGCT | 90 |
| $S_3$ | AAGCGTTCACTTCGGAAAACTATGGCTCGCCT | 91 |
| $S_4$ | AAGGCGAGCCATAGTAAAGGATTGACGACGCT | 92 |
| Sequence set 4-9 numbering | Optimized sequence | |
| $S_1$ | ATGTGGCGACAATCCAAATGAGCAAGCCTCCA | 93 |

(continued)

| Sequence set 4-9 numbering | Optimized sequence | |
|---|---|---|
| S₂ | ATGGAGGCTTGCTCAAAACCGAAGACGCTGTT | 94 |
| S₃ | AAACAGCGTCTTCGGAAAACTATCGTGCGGCA | 95 |
| S₄ | ATGCCGCACGATAGTAAAGGATTGTCGCCACA | 96 |
| Sequence set 4-10 numbering | Optimized sequence | |
| S₁ | ATGCTGCCACAATCCAAATGAGCCTGGAACCA | 97 |
| S₂ | ATGGTTCCAGGCTCAAAACCGAACGCAGTCAT | 98 |
| S₃ | AATGACTGCGTTCGGAAAACTATCGCCGCTCT | 99 |
| S₄ | AAGAGCGGCGATAGTAAAGGATTGTGGCAGCA | 100 |
| Sequence set 4-11 numbering | Optimized sequence | |
| S₁ | ATGCGTCGTCAATCCAAATGAGCTTGGCAAGG | 101 |
| S₂ | ACCTTGCCAAGCTCAAAACCGAACGTGCTGTT | 102 |
| S₃ | AAACAGCACGTTCGGAAAACTATGGAGCGGCT | 103 |
| S₄ | AAGCCGCTCCATAGTAAAGGATTGACGACGCA | 104 |
| Sequence set 4-12 numbering | Optimized sequence | |
| S₁ | AACTGCCAGCAATCCAAATGAGCCTCGTTCCA | 105 |
| S₂ | ATGGAACGAGGCTCAAAACCGAAGTTGGCAGT | 106 |
| S₃ | AACTGCCAACTTCGGAAAACTATCGCCGCTTG | 107 |
| S₄ | ACAAGCGGCGATAGTAAAGGATTGCTGGCAGT | 108 |
| Sequence set 4-13 numbering | Optimized sequence | |
| S₁ | ATGCGTCGTCAATCCAAATGAGCCTCCAGGTT | 109 |

(continued)

| Sequence set 4-13 numbering | Optimized sequence | |
|---|---|---|
| $S_2$ | AAACCTGGAGGCTCAAAACCGAATGACACGCT | 110 |
| $S_3$ | AAGCGTGTCATTCGGAAAACTATGGCGGCAGT | 111 |
| $S_4$ | AACTGCCGCCATAGTAAAGGATTGACGACGCA | 112 |
| Sequence set 4-14 numbering | Optimized sequence | |
| $S_1$ | AAGCGTCGTGAATCCAAATGAGCCATCGTCCA | 113 |
| $S_2$ | ATGGACGATGGCTCAAAACCGAATGTGCTGGT | 114 |
| $S_3$ | AACCAGCACATTCGGAAAACTATGCGGCAACC | 115 |
| $S_4$ | AGGTTGCCGCATAGTAAAGGATTCACGACGCT | 116 |
| Sequence set 4-15 numbering | Optimized sequence | |
| $S_1$ | ATTGCCAGGATGCTGAATCACGGTCGGACA | 117 |
| $S_2$ | ATGTCCGACCGTGATAGTCGCAGAAGGCAT | 118 |
| $S_3$ | AATGCCTTCTGCGACATAGTACAACGCCGC | 119 |
| $S_4$ | AGCGGCGTTGTACTAACAGCATCCTGGCAA | 120 |
| Sequence set 4-16 numbering | Optimized sequence | |
| $S_1$ | AGGCGATCACAATCCAAATGAGCGTGTTACGG | 121 |
| $S_2$ | ACCGTAACACGCTCAAAACCGAAGTGCCAATT | 122 |
| $S_3$ | AAATTGGCACTTCGGAAAACTATGCGGCTGCT | 123 |
| $S_4$ | AAGCAGCCGCATAGTAAAGGATTGTGATCGCC | 124 |
| Sequence set 4-17 numbering | Optimized sequence | |
| $S_1$ | ATGGTCCAACACGCTAAGCCTCACCGTCTT | 125 |

(continued)

| Sequence set 4-17 numbering | Optimized sequence | |
|---|---|---|
| $S_2$ | <u>A</u>AAGACGGTGAGGCT<u>A</u>TCGCACAACCTGGT | 126 |
| $S_3$ | <u>A</u>ACCAGGTTGTGCGA<u>A</u>TCGGAGTGGCAGAA | 127 |
| $S_4$ | <u>A</u>TTCTGCCACTCCGA<u>A</u>AGCGTGTTGGACCA | 128 |
| Sequence set 4-18 numbering | Optimized sequence | |
| $S_1$ | <u>A</u>ACCTTGGTGTGCGA<u>A</u>ACTCCTGGCAGCAA | 129 |
| $S_2$ | <u>A</u>TTGCTGCCAGGAGT<u>A</u>AGCGTGTGGTTCCA | 130 |
| $S_3$ | <u>A</u>TGGAACCACACGCT<u>A</u>TGAGGACCGTCGTT | 131 |
| $S_4$ | <u>A</u>AACGACGGTCCTCA<u>A</u>TCGCACACCAAGGT | 132 |
| Sequence set 4-19 numbering | Optimized sequence | |
| $S_1$ | <u>A</u>TGCCAAGTCCGAGA<u>A</u>TGCTGCGAACTGGT | 133 |
| $S_2$ | <u>A</u>ACCAGTTCGCAGCA<u>A</u>AGAGCCTGAACCGT | 134 |
| $S_3$ | <u>A</u>ACGGTTCAGGCTCT<u>A</u>ACGACGCTTGACCA | 135 |
| $S_4$ | <u>A</u>TGGTCAAGCGTCGT<u>A</u>TCTCGGACTTGGCA | 136 |
| Sequence set 4-20 numbering | Optimized sequence | |
| $S_1$ | <u>A</u>AGCAGCCTCGTTGA<u>A</u>TCGCCAAGACACCT | 137 |
| $S_2$ | <u>A</u>AGGTGTCTTGGCGA<u>A</u>AGTTGCTCCGACGA | 138 |
| $S_3$ | <u>A</u>TCGTCGGAGCAACT<u>A</u>AGCGGTTCTGTGGA | 139 |
| $S_4$ | <u>A</u>TCCACAGAACCGCT<u>A</u>TCAACGAGGCTGCT | 140 |

(S3) a nucleic acid single strand sequence for forming a pentamer complex based on the complementary nucleic acid backbone:

| Sequence set 5-1 numbering | Optimized sequence | SEQ ID NO: |
|---|---|---|
| $S_1$ | ATCAGGCGACCTCTTAAAACCACCATCGT TGC | 141 |
| $S_2$ | AGCAACGATGGTGGTAAAAATCCAAATGA GCGTGTTACGG | 142 |
| $S_3$ | ACCGTAACACGCTCAAAACCGAAGTGCCA ATT | 143 |
| $S_4$ | AAATTGGCACTTCGGAAAACTATGCGGCT GCT | 144 |
| $S_5$ | AAGCAGCCGCATAGTAAAGGATTAAAAA GAGGTCGCCTGA | 145 |
| Sequence set 5-2 numbering | Optimized sequence | |
| $S_1$ | AGGCGACGATGTCTTAAAACCTGGTTGCT GGA | 146 |
| $S_2$ | ATCCAGCAACCAGGTAAAAATCCAAATGA GCGTGTTACGG | 147 |
| $S_3$ | ACCGTAACACGCTCAAAACCGAAGTGCCA ATT | 148 |
| $S_4$ | AAATTGGCACTTCGGAAAACTATGCGGCT GCT | 149 |
| $S_5$ | AAGCAGCCGCATAGTAAAGGATTAAAAA GACATCGTCGCC | 150 |
| Sequence set 5-3 numbering | Optimized sequence | |
| $S_1$ | ATGGAACCTGGTGCTAAATGCTCGCCTGT CAA | 151 |
| $S_2$ | ATTGACAGGCGAGCAAAAAATCCAAATG AGCGTGTTACGG | 152 |
| $S_3$ | ACCGTAACACGCTCAAAACCGAAGTGCCA ATT | 153 |
| $S_4$ | AAATTGGCACTTCGGAAAACTATGCGGCT GCT | 154 |
| $S_5$ | AAGCAGCCGCATAGTAAAGGATTAAAAG CACCAGGTTCCA | 155 |
| Sequence set 5-4 numbering | Optimized sequence | |
| $S_1$ | ATGGTCAGGCGACTTAAAAGGACGAGGTT GCT | 156 |

(continued)

| Sequence set 5-4 numbering | Optimized sequence | |
|---|---|---|
| $S_2$ | AAGCAACCTCGTCCTAAAAATCCAAATGAGCGTGTTACGG | 157 |
| $S_3$ | ACCGTAACACGCTCAAAACCGAAGTGCCAATT | 158 |
| $S_4$ | AAATTGGCACTTCGGAAACTATGCGGCTGCT | 159 |
| $S_5$ | AAGCAGCCGCATAGTAAAGGATTAAAAAGTCGCCTGACCA | 160 |
| Sequence set 5-5 numbering | Optimized sequence | |
| $S_1$ | ATGCTGGACCACCTTAAATCAGATGGAGGCGA | 161 |
| $S_2$ | ATCGCCTCCATCTGAAAAAATCCAAATGAGCGTGTTACGG | 162 |
| $S_3$ | ACCGTAACACGCTCAAAACCGAAGTGCCAATT | 163 |
| $S_4$ | AAATTGGCACTTCGGAAACTATGCGGCTGCT | 164 |
| $S_5$ | AAGCAGCCGCATAGTAAAGGATTAAAAAGGTGGTCCAGCA | 165 |
| Sequence set 5-6 numbering | Optimized sequence | |
| $S_1$ | AAACGTCCAGGAGCTAAATCTCGTCGCCTGAA | 166 |
| $S_2$ | ATTCAGGCGACGAGAAAAAATCCAAATGAGCGTGTTACGG | 167 |
| $S_3$ | ACCGTAACACGCTCAAAACCGAAGTGCCAATT | 168 |
| $S_4$ | AAATTGGCACTTCGGAAACTATGCGGCTGCT | 169 |
| $S_5$ | AAGCAGCCGCATAGTAAAGGATTAAAAGCTCCTGGACGTT | 170 |
| Sequence set 5-7 numbering | Optimized sequence | |
| $S_1$ | ACCACGACCATTGCTAAAACTTCAGGCGACG | 171 |
| $S_2$ | ACGTCGCCTGAAGTTAAAAATCCAAATGAGCGTGTTACGG | 172 |

(continued)

| Sequence set 5-7 numbering | Optimized sequence | |
|---|---|---|
| $S_3$ | ACCGTAACACGCTCAAAACCGAAGTGCCAATT | 173 |
| $S_4$ | AAATTGGCACTTCGGAAACTATGCGGCTGCT | 174 |
| $S_5$ | AAGCAGCCGCATAGTAAAGGATTAAAAGCAATGGTCGTGG | 175 |
| Sequence set 5-8 numbering | Optimized sequence | |
| $S_1$ | AAGGCGAGGTCTTCAAAATGGTTGCTGGACGA | 176 |
| $S_2$ | ATCGTCCAGCAACCAAAAAATCCAAATGAGCGTGTTACGG | 177 |
| $S_3$ | ACCGTAACACGCTCAAAACCGAAGTGCCAATT | 178 |
| $S_4$ | AAATTGGCACTTCGGAAACTATGCGGCTGCT | 179 |
| $S_5$ | AAGCAGCCGCATAGTAAAGGATTAAATGAAGACCTCGCCT | 180 |
| Sequence set 5-9 numbering | Optimized sequence | |
| $S_1$ | ATCAAGGCGACCAGTAAAAAGCTCCTCGACGA | 181 |
| $S_2$ | ATCGTCGAGGAGCTTAAAAATCCAAATGAGCGTGTTACGG | 182 |
| $S_3$ | ACCGTAACACGCTCAAAACCGAAGTGCCAATT | 183 |
| $S_4$ | AAATTGGCACTTCGGAAACTATGCGGCTGCT | 184 |
| $S_5$ | AAGCAGCCGCATAGTAAAGGATTAAAACTGGTCGCCTTGA | 185 |
| Sequence set 5-10 numbering | Optimized sequence | |
| $S_1$ | ATTCAGGCGACTCCTAAAAGCACGACGATGGT | 186 |
| $S_2$ | AACCATCGTCGTGCTAAAAATCCAAATGAGCGTGTTACGG | 187 |
| $S_3$ | ACCGTAACACGCTCAAAACCGAAGTGCCAATT | 188 |

(continued)

| Sequence set 5-10 numbering | Optimized sequence | |
|---|---|---|
| S₄ | AAATTGGCACTTCGGAAAACTATGCGGCT GCT | 189 |
| S₅ | AAGCAGCCGCATAGTAAAGGATTAAAAG GAGTCGCCTGAA | 190 |
| Sequence set 5-11 numbering | Optimized sequence | |
| S₁ | AAGCACCTGCAATCCAAATCGCCAGGACA AGT | 191 |
| S₂ | AACTTGTCCTGGCGAAAATGAGCAACCAT GCC | 192 |
| S₃ | AGGCATGGTTGCTCAAAACCGAACGTCGT GAT | 193 |
| S₄ | AATCACGACGTTCGGAAAACTATGGAGCG GCT | 194 |
| S₅ | AAGCCGCTCCATAGTAAAGGATTGCAGGT GCT | 195 |
| Sequence set 5-12 numbering | Optimized sequence | |
| S₁ | AACCTGCTGCAATCCAAATCGCCACCTCA AGA | 196 |
| S₂ | ATCTTGAGGTGGCGAAAATGAGCCTGGAC GTT | 197 |
| S₃ | AAACGTCCAGGCTCAAAACCGAACTGGTG CTT | 198 |
| S₄ | AAAGCACCAGTTCGGAAAACTATGCCGCT CCT | 199 |
| S₅ | AAGGAGCGGCATAGTAAAGGATTGCAGC AGGT | 200 |
| Sequence set 5-13 numbering | Optimized sequence | |
| S₁ | AAGCTGGTGCAATCCAAATCGCCTCCTGA CAA | 201 |
| S₂ | ATTGTCAGGAGGCGAAAATGAGCAAGGTT GGC | 202 |
| S₃ | AGCCAACCTTGCTCAAAACCGAACGCAGA TGT | 203 |
| S₄ | AACATCTGCGTTCGGAAAACTATGGAGCG GCA | 204 |

(continued)

| Sequence set 5-13 numbering | Optimized sequence | |
|---|---|---|
| $S_5$ | <u>A</u>TGCCGCTCCATAGT<u>AAA</u>GGATTGCACCAGCT | 205 |
| Sequence set 5-14 numbering | Optimized sequence | |
| $S_1$ | <u>A</u>TGCACGCACAATCC<u>AAA</u>TCGCCATCAGAGGT | 206 |
| $S_2$ | <u>A</u>ACCTCTGATGGCGA<u>AAA</u>TGAGCTGCCTCCAT | 207 |
| $S_3$ | <u>A</u>ATGGAGGCAGCTCA<u>AAA</u>CCGAACGTCGTCAT | 208 |
| $S_4$ | <u>A</u>ATGACGACGTTCGG<u>AAA</u>ACTATCGAGCGGCT | 209 |
| $S_5$ | <u>A</u>AGCCGCTCGATAGT<u>AAA</u>GGATTGTGCGTGCA | 210 |
| Sequence set 5-15 numbering | Optimized sequence | |
| $S_1$ | <u>A</u>AGCGTCGTGAATCC<u>AAA</u>TCGCCATCAGACCA | 211 |
| $S_2$ | <u>A</u>TGGTCTGATGGCGA<u>AAA</u>TGAGCAAGGCTCGT | 212 |
| $S_3$ | <u>A</u>ACGAGCCTTGCTCA<u>AAA</u>CCGAACCAGCTTGT | 213 |
| $S_4$ | <u>A</u>ACAAGCTGGTTCGG<u>AAA</u>ACTATGCGGCAGGT | 214 |
| $S_5$ | <u>A</u>ACCTGCCGCATAGT<u>AAA</u>GGATTCACGACGCT | 215 |
| Sequence set 5-16 numbering | Optimized sequence | |
| $S_1$ | <u>A</u>TCAGCACGCAATCC<u>AAA</u>TCGCCAGTTCAACC | 216 |
| $S_2$ | <u>A</u>GGTTGAACTGGCGA<u>AAA</u>TGAGCAAGCAGGCT | 217 |
| $S_3$ | <u>A</u>AGCCTGCTTGCTCA<u>AAA</u>CCGAACGTGGTGTT | 218 |
| $S_4$ | <u>A</u>AACACCACGTTCGG<u>AAA</u>ACTATGGAGCGGCA | 219 |
| $S_5$ | <u>A</u>TGCCGCTCCATAGT<u>AAA</u>GGATTGCGTGCTGA | 220 |

(continued)

| Sequence set 5-17 numbering | Optimized sequence | |
|---|---|---|
| $S_1$ | AAGCTGCACCAATCCAAATCGCCAGAAGGTCA | 221 |
| $S_2$ | ATGACCTTCTGGCGAAAATGAGCACGACGCAT | 222 |
| $S_3$ | AATGCGTCGTGCTCAAAACCGAACAACCTGCT | 223 |
| S4 | AAGCAGGTTGTTCGGAAAACTATGGAGCGGCA | 224 |
| $S_5$ | ATGCCGCTCCATAGTAAAGGATTGGTGCAGCT | 225 |
| Sequence set 5-18 numbering | Optimized sequence | |
| $S_1$ | AACGCTCGTCAATCCAAATCGCCTCAGGACAA | 226 |
| $S_2$ | ATTGTCCTGAGGCGAAAATGAGCCAACGACCT | 227 |
| $S_3$ | AAGGTCGTTGGCTCAAAACCGAAGCTGGTGTT | 228 |
| $S_4$ | AAACACCAGCTTCGGAAAACTATGCCGCACCT | 229 |
| $S_5$ | AAGGTGCGGCATAGTAAAGGATTGACGAGCGT | 230 |
| Sequence set 5-19 numbering | Optimized sequence | |
| $S_1$ | AAGTGCGTCGAATCCAAATCGCCAAGACCTCA | 231 |
| $S_2$ | ATGAGGTCTTGGCGAAAATGAGCAGGCTGGAA | 232 |
| $S_3$ | ATTCCAGCCTGCTCAAAACCGAAGCAACGTGT | 233 |
| $S_4$ | AACACGTTGCTTCGGAAAACTATGCCGCTCCT | 234 |
| $S_5$ | AAGGAGCGGCATAGTAAAGGATTCGACGCACT | 235 |
| Sequence set 5-20 numbering | Optimized sequence | |
| $S_1$ | ATCACGCAGCAATCCAAATCGCCATCACAACG | 236 |

(continued)

| Sequence set 5-20 numbering | Optimized sequence | |
|---|---|---|
| $S_2$ | <u>A</u>CGTTGTGATGGCGA<u>AAA</u>TGAGCACGAGC CTT | 237 |
| $S_3$ | <u>A</u>AAGGCTCGTGCTCA<u>AAA</u>CCGAAGGTTGC ACT | 238 |
| $S_4$ | <u>A</u>AGTGCAACCTTCGG<u>AAA</u>ACTATGCCGCT CCA | 239 |
| $S_5$ | <u>A</u>TGGAGCGGCATAGT<u>AAA</u>GGATTGCTGCG TGA | 240 |

(A) Trimer

(B) Tetramer

(C) Pentamer

# Figure 1

# Figure 2

S1. 5' **ACCACCGTGTATGACCT**AAA**AGTGACAGCACATCGC** 3'
S2. 5' **AGCGATGTGCTGTCACT**AAA**ACAGGCTCTACGAGGA** 3'
S3. 5' **ATCCTCGTAGAGCCTGT**AAA**AGGTCATACACGGTGG** 3'

**Figure 3**

**Figure 4**

S1. 5' AGGCGATCACAATCCAAATGAGCGTGTTACGG 3'
S2. 5' ACCGTAACACGCTCAAAACCGAAGTGCCAATT 3'
S3. 5' AAATTGGCACTTCGGAAAACTATGCGGCTGCT 3'
S4. 5' AAGCAGCCGCATAGTAAAGGATTGTGATCGCC 3'

**Figure 5**

**i-th count (score is counted every 15 iterations)**

**Figure 6**

**Figure 7**

**Figure 8**

Figure 9

Figure 10

S1. 5' ATCACGCAGCAATCCAAATCGCCATCACAACG 3'
S2. 5' ACGTTGTGATGGCGAAAATGAGCACGAGCCTT 3'
S3. 5' AAAGGCTCGTGCTCAAAACCGAAGGTTGCACT 3'
S4. 5' AAGTGCAACCTTCGGAAAACTATGCCGCTCCA 3'
S5. 5' ATGGAGCGGCATAGTAAAGGATTGCTGCGTGA 3'

**Tetramer core structure**
S1. 5' A...AATCCAAATGAGC... 3'
S2. 5' A...GCTCAAAACCGAA... 3'
S3. 5' A...TTCGGAAAACTAT... 3'
S4. 5' A...ATAGTAAAGGATT... 3'

## Figure 11

## Figure 12

Figure 13

Figure 14

**Figure 15**

**Figure 16**

**Figure 17**

**Figure 18**

1: Marker
2: PMO1
3: SM(PEG)₂-PMO1
4: SM(PEG)₂-PMO1 and anti-HAS Nb were coupled
   at room temperature for 2 hours

**Figure 19**

**Figure 20**

**Figure 21**

1. Anti-HSA Nb-PMO1
2. Anti-HSA Nb-PMO2
3. Anti-HSA Nb-PMO3
4. pmo-NAPPA4-HSA(1,2,3)

5. pmo-NAPPA4-HSA(1)

**Figure 22**

| Name | EC50 (nM) |
|------|-----------|
| Anti-HSA Nb | 0.577 |
| Anti-HSA Nb-PMO1 | 0.391 |
| pmo-NAPPA4-HSA(1) | 0.529 |

**Figure 23**

**Figure 24**

## INTERNATIONAL SEARCH REPORT

| International application No. |
|---|
| **PCT/CN2021/133254** |

### A. CLASSIFICATION OF SUBJECT MATTER

C40B 40/06(2006.01)i; A61K 38/02(2006.01)i; A61K 38/37(2006.01)i; A61K 39/395(2006.01)i; A61K 47/54(2017.01)i

According to International Patent Classification (IPC) or to both national classification and IPC

### B. FIELDS SEARCHED

Minimum documentation searched (classification system followed by classification symbols)

C40B;A61K

Documentation searched other than minimum documentation to the extent that such documents are included in the fields searched

Electronic data base consulted during the international search (name of data base and, where practicable, search terms used)

DWPI, VEN, SIPOABS, CNABS, CNTXT, USTXT, EPTXT, WOTXT, 万方数据库, CNKI, PubMed, ISI web of Knowledge, GenBank, EBI, STN: 核酸, 温度, 集合, 解离温度, 互配, 计算, 核酸配对, GC比, CG比, 配对, 退火算法, 参数, 阈值, 解离, 退火, 自由能, 阈值, 核酸-蛋白, 核酸-多肽, 核酸-药物, 多聚体, 杂交, 安升, 周界文, 闫长青, 曹婵, 杨凡, 周柳娟, nucleic acid-protein, nucleic acid-peptide?, multimer, monomer, protein drug, component, double strand?, pair?, 序列表序列

### C. DOCUMENTS CONSIDERED TO BE RELEVANT

| Category* | Citation of document, with indication, where appropriate, of the relevant passages | Relevant to claim No. |
|---|---|---|
| PX | CN 112746331 A (ANSHENG (SHANGHAI) MEDICINE TECHNOLOGY CO., LTD.) 04 May 2021 (2021-05-04) <br> description paragraphs 7-122, description, figure 2 | 1-15 |
| X | WO 2018205755 A1 (ANSHENG (SHANGHAI) MEDICINE TECHNOLOGY CO., LTD.) 15 November 2018 (2018-11-15) <br> claims 1-10, description pages 2-7, description figures 1-2 | 1-4, 9 |
| Y | WO 2018205755 A1 (ANSHENG (SHANGHAI) MEDICINE TECHNOLOGY CO., LTD.) 15 November 2018 (2018-11-15) <br> claims 1-10, description pages 2-7, description figures 1-2 | 5-8, 10-15 |
| X | US 2003027194 A1 (MARKUS KURZ et al.) 06 February 2003 (2003-02-06) <br> claims 1-32, description figures 1-3 | 1-4, 9 |
| Y | US 2003027194 A1 (MARKUS KURZ et al.) 06 February 2003 (2003-02-06) <br> claims 1-32, description figures 1-3 | 5-8, 10-15 |

☑ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **21 January 2021** | **23 February 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2021/133254** |

**C. DOCUMENTS CONSIDERED TO BE RELEVANT**

| Category* | Citation of document, with indication, where appropriate, of the relevant passages | Relevant to claim No. |
|---|---|---|
| Y | 张凯 等 (ZHANG, Kai et al.). "基于多目标进化策略算法的DNA核酸编码设计 (A Multiobjective Evolution Strategy Algorithm for DNA Sequence Design)" 电子与信息学报 (Journal of Electronics & Information Technology), Vol. 42, No. 6, 30 June 2020 (2020-06-30), pp. 1366-1373 | 5-8, 10-15 |
| A | US 5561043 A (TRUSTEES OF BOSTON UNIVERSITY) 01 October 1996 (1996-10-01) entire document | 1-15 |
| A | US 2011171639 A1 (EISAI R&D MANAGEMENT CO., LTD.) 14 July 2011 (2011-07-14) entire document | 1-15 |
| A | CN 101866388 A (PEKING UNIVERSITY) 20 October 2010 (2010-10-20) entire document | 1-15 |
| A | US 5124246 A (CHIRON CORPORATION) 23 June 1992 (1992-06-23) entire document | 1-15 |
| A | 吕鸣 等 (LYU, Ming et al.). "一种基于关键路径法的DNA计算用寡核苷酸序列设计算法 (A Sequences Designing Algorithm for DNA Computation Based on Critical Path Method)" 生物信息学 (China Journal of Bioinformatics), Vol. 5, No. 2, 30 June 2007 (2007-06-30), pp. 62-66 | 1-15 |

Form PCT/ISA/210 (second sheet) (January 2015)

## INTERNATIONAL SEARCH REPORT

| International application No. |
| --- |
| **PCT/CN2021/133254** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

# EP 4 253 609 A1

## INTERNATIONAL SEARCH REPORT
### Information on patent family members

| International application No. |
| --- |
| **PCT/CN2021/133254** |

| Patent document cited in search report | | | Publication date (day/month/year) | Patent family member(s) | | | Publication date (day/month/year) |
| --- | --- | --- | --- | --- | --- | --- | --- |
| CN | 112746331 | A | 04 May 2021 | None | | | |
| WO | 2018205755 | A1 | 15 November 2018 | CN | 110621814 | A | 27 December 2019 |
| | | | | US | 2020376070 | A1 | 03 December 2020 |
| | | | | CA | 3062962 | A1 | 02 December 2019 |
| | | | | KR | 20200004869 | A | 14 January 2020 |
| | | | | EP | 3623503 | A1 | 18 March 2020 |
| | | | | EP | 3623503 | A4 | 24 February 2021 |
| | | | | AU | 2018266091 | A1 | 12 December 2019 |
| | | | | AU | 2018266091 | B2 | 02 July 2020 |
| | | | | JP | 2020519696 | A | 02 July 2020 |
| | | | | JP | 6927618 | B2 | 01 September 2021 |
| | | | | CN | 108866635 | A | 23 November 2018 |
| US | 2003027194 | A1 | 06 February 2003 | EP | 1419274 | A1 | 19 May 2004 |
| | | | | EP | 1419274 | A4 | 08 February 2006 |
| | | | | AU | 2002324571 | A1 | 17 February 2003 |
| | | | | CA | 2456105 | A1 | 13 February 2003 |
| | | | | WO | 03012146 | A1 | 13 February 2003 |
| | | | | JP | 2004537312 | A | 16 December 2004 |
| US | 5561043 | A | 01 October 1996 | WO | 9520320 | A1 | 03 August 1995 |
| | | | | JP | H09511641 | A | 25 November 1997 |
| | | | | US | 5965133 | A | 12 October 1999 |
| | | | | EP | 0744894 | A1 | 04 December 1996 |
| | | | | EP | 0744894 | A4 | 12 January 2000 |
| | | | | AU | 1730595 | A | 15 August 1995 |
| US | 2011171639 | A1 | 14 July 2011 | JP | WO2009057702 | A1 | 10 March 2011 |
| | | | | EP | 2213751 | A1 | 04 August 2010 |
| | | | | WO | 2009057702 | A1 | 07 May 2009 |
| CN | 101866388 | A | 20 October 2010 | CN | 101866388 | B | 04 July 2012 |
| US | 5124246 | A | 23 June 1992 | WO | 9013667 | A1 | 15 November 1990 |
| | | | | ES | 2087153 | T3 | 16 July 1996 |
| | | | | AT | 138691 | T | 15 June 1996 |
| | | | | KR | 920701480 | A | 11 August 1992 |
| | | | | KR | 0156007 | B1 | 15 October 1998 |
| | | | | CA | 2014855 | A1 | 18 October 1990 |
| | | | | DE | 69027211 | D1 | 04 July 1996 |
| | | | | DE | 69027211 | T2 | 17 October 1996 |
| | | | | AU | 5563690 | A | 29 November 1990 |
| | | | | AU | 641393 | B2 | 23 September 1993 |
| | | | | EP | 0469066 | A1 | 05 February 1992 |
| | | | | EP | 0469066 | A4 | 08 July 1992 |
| | | | | EP | 0469066 | B1 | 29 May 1996 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAMBROOK et al.** Molecular Cloning: A Laboratory Manual. Cold Spring Harbor Laboratory Press, 1989